# EUROPEAN PATENT APPLICATION

(11) **EP 4 674 965 A1**
(43) Date of publication of application: **07.01.2026**
(21) Application number: 24763253.2
(22) Date of filing: 01.03.2024
(51) Int. Cl.: C12N 15/113, A61K 31/7088, A61K 31/7125, A61P 3/00

(54) **SIRNA TARGETING INHIBIN ?E, SIRNA CONJUGATE, AND MEDICAL USE THEREOF**

(30) Priority: 02.03.2023 CN 202310194029; 19.04.2023 CN 202310420703; 28.09.2023 CN 202311271773; 08.01.2024 CN 202410023838
(71) Applicant: Tuojie Biotech (Shanghai) Co., Ltd., Shanghai 201203 (CN)
(72) Inventor: YAN, Cuicui, Shanghai 201203 (CN); WEN, Yuhao, Shanghai 201203 (CN); LIN, Xiaoyan, Shanghai 201203 (CN); LI, Yunfei, Shanghai 201203 (CN)
(74) Representative: Potter Clarkson
(86) International application number: PCT/CN2024/079576
(87) International publication number: WO 2024/179573

(57) **Abstract**

The invention relates to an siRNA targeting inhibin βE, an siRNA conjugate, and medical application thereof; and specifically relates to an siRNA targeting INHBE, an siRNA conjugate, a composition, and a medical use thereof. The invention further relates to a pharmaceutical composition, a cell, or a reagent test kit comprising the siRNA, and to a method for using the siRNA and the siRNA conjugate in treatment and/or prevention for a subject suffering from a related disorder.

## Description

The present disclosure claims priority to Chinese Patent Application No. 202310194029.7 filed on March 2, 2023, Chinese Patent Application No. 202310420703.9 filed on April 19, 2023, Chinese Patent Application No. 202311271773.9 filed on September 28, 2023, and Chinese Patent Application No. 202410023838.6 filed on January 8, 2024, which are incorporated herein by reference in their entirety.

### TECHNICAL FIELD

The present disclosure pertains to the field of biomedicine, and particularly relates to an siRNA targeting the inhibin βE (INHBE) gene, a conjugate, a composition, and pharmaceutical use thereof.

### BACKGROUND

The inhibin βE (INHBE) gene, one member of the TGF-β (transforming growth factor-β) superfamily proteins, is involved in the regulation of various cellular processes, including cell proliferation, apoptosis, immune responses, and hormone secretion. Inhibins are responsible for inhibiting the secretion of follicle-stimulating hormone from the pituitary gland, and may be involved in the regulation of many different functions, such as hypothalamic and pituitary hormone secretion, gonadal hormone secretion, germ cell development and maturation, red blood cell differentiation, insulin secretion, nerve cell survival, embryo axial development, or bone growth. In addition, INHBE may be up-regulated under endoplasmic reticulum stress conditions, and thus the protein may inhibit the proliferation and growth of cells in the pancreas and the liver.

Body fat distribution is an important determination criterion for cardiovascular and metabolic diseases. A specific one among various body fat distributions is apt to give rise to serious cardiovascular or metabolic diseases, and is characterized by the distribution of more fat on the waist and/or the accumulation of less fat in the hip, resulting in a relatively high waist-hip ratio (WHR). Metabolic diseases caused by abnormal body fat distribution include: type 2 diabetes, hyperlipidemia, dyslipidemia (elevated or changed circulation levels of low-density lipoprotein cholesterol LDL-C, triglyceride, very low-density lipoprotein cholesterol VLDL-C, apolipoprotein B, or other lipid fractions), obesity (particularly abdominal obesity), lipodystrophy, insulin resistance, nonalcoholic steatohepatitis, hypertension and/or high blood pressure, high blood sugar or glucose or hyperglycemia, metabolic syndrome, coronary artery disease, other atherosclerotic disorders, and the like.

At present, methods for treating metabolic diseases mainly include lifestyle modifications, dietary adjustments, physical exercise, and administration of lipid-lowering drugs (e.g., statins). However, these methods have relatively poor effects, and the administration of lipid-lowering drugs may bring some side effects. RNA interference (RNAi; siRNA) is an effective approach to silence gene expression, which can specifically degrade mRNAs of target genes (e.g., INHBE) associated with metabolic diseases through a post-transcriptional regulatory mechanism, thereby effectively inhibiting the expression of the target genes and achieving the purpose of effectively treating such diseases.

### SUMMARY

WO2022028462A relates to an siRNA with an anti-off-target modification, which is incorporated herein by reference in its entirety.

WO2023274395A relates to a nucleic acid ligand and a conjugate thereof, which is incorporated herein by reference in its entirety.

The present disclosure provides an siRNA targeting INHBE.

In some embodiments, the present disclosure provides an siRNA, which comprises a sense strand and an antisense strand forming a double-stranded region, wherein
the sense strand comprises at least 15 (e.g., 16, 17, 18, 19, 20, or 21) contiguous nucleotides and differs from any one of the nucleotide sequences of SEQ ID NO: 1 to SEQ ID NO: 9 by no more than 3 (e.g., 0, 1, 2, or 3) nucleotides;
the antisense strand comprises at least 15 (e.g., 16, 17, 18, 19, 20, 21, 22, or 23) contiguous nucleotides and differs from any one of the nucleotide sequences of SEQ ID NO: 10 to SEQ ID NO: 18 by no more than 3 (e.g., 0, 1, 2, or 3) nucleotides.

In some embodiments, the antisense strand is at least partially reverse complementary to a target sequence to mediate RNA interference. In some embodiments, there are no more than 5, no more than 4, no more than 3, no more than 2, or no more than 1 mismatch between the antisense strand and the target sequence. In some embodiments, the antisense strand is fully reverse complementary to the target sequence.

In some embodiments, the sense strand is at least partially reverse complementary to the antisense strand to form a double-stranded region. In some embodiments, there are no more than 5, no more than 4, no more than 3, no more than 2, or no more than 1 mismatch between the sense strand and the antisense strand. In some embodiments, the sense strand is fully reverse complementary to the antisense strand.

In some embodiments, the siRNA of the present disclosure comprises one or two blunt ends.

In some specific embodiments, each strand of the siRNA independently comprises an overhang having 1 to 2 unpaired nucleotides.

In some embodiments, the siRNA of the present disclosure comprises an overhang at the 3' end of the antisense strand.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides (e.g., 19, 20, 21, 22, or 23 nucleotides).

In some embodiments, the sense strand and the antisense strand are identical or different in length; the sense strand is 19-23 nucleotides in length, and the antisense strand is 19-26 nucleotides in length. The length ratio of the sense strand to the antisense strand of the siRNA provided by the present disclosure may be 19/19, 19/20, 19/21, 19/22, 19/23, 19/24, 19/25, 19/26, 20/19, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26.

In some embodiments, the length ratio of the sense strand to the antisense strand of the siRNA is 19/21, 21/21, 21/23, or 23/25. In some embodiments, the length ratio of the sense strand to the antisense strand of the siRNA is 19/21, 21/21, or 21/23. In some embodiments, the length ratio of the sense strand to the antisense strand of the siRNA is 19/21.

In some embodiments, the sense strand comprises at least 15 contiguous nucleotides and differs from any one of the nucleotide sequences of SEQ ID NO: 1 to SEQ ID NO: 9 and SEQ ID NO: 19 to SEQ ID NO: 122 by no more than 2 nucleotides. In some embodiments, the nucleotide sequence differs by no more than 1 nucleotide; in some embodiments, the difference is 1 nucleotide.

In some embodiments, the antisense strand comprises at least 15 contiguous nucleotide sequences and differs from any one of the nucleotide sequences of SEQ ID NO: 10 to SEQ ID NO: 18 and SEQ ID NO: 123 to SEQ ID NO: 226 by no more than 2 nucleotides; in some embodiments, the nucleotide sequence differs by no more than 1 nucleotide; in some embodiments, the difference is 1 nucleotide.

In some embodiments, the sense strand comprises at least 15 contiguous nucleotides in any one of the nucleotide sequences of SEQ ID NO: 1 to SEQ ID NO: 9 and SEQ ID NO: 19 to SEQ ID NO: 122. In some embodiments, the sense strand comprises at least 16 contiguous nucleotides in any one of the nucleotide sequences of SEQ ID NO: 1 to SEQ ID NO: 9 and SEQ ID NO: 19 to SEQ ID NO: 122. In some embodiments, the sense strand comprises at least 17 contiguous nucleotides in any one of the nucleotide sequences of SEQ ID NO: 1 to SEQ ID NO: 9 and SEQ ID NO: 19 to SEQ ID NO: 122. In some embodiments, the sense strand comprises at least 19 contiguous nucleotides in any one of the nucleotide sequences of SEQ ID NO: 1 to SEQ ID NO: 9 and SEQ ID NO: 19 to SEQ ID NO: 122.

In some embodiments, the antisense strand comprises at least 15 contiguous nucleotides in any one of the nucleotide sequences of SEQ ID NO: 10 to SEQ ID NO: 18 and SEQ ID NO: 123 to SEQ ID NO: 226. In some embodiments, the antisense strand comprises at least 17 contiguous nucleotides in any one of the nucleotide sequences of SEQ ID NO: 10 to SEQ ID NO: 18 and SEQ ID NO: 123 to SEQ ID NO: 226. In some embodiments, the antisense strand comprises at least 19 contiguous nucleotides in any one of the nucleotide sequences of SEQ ID NO: 10 to SEQ ID NO: 18 and SEQ ID NO: 123 to SEQ ID NO: 226. In some embodiments, the antisense strand comprises at least 20 contiguous nucleotides in any one of the nucleotide sequences of SEQ ID NO: 10 to SEQ ID NO: 18 and SEQ ID NO: 123 to SEQ ID NO: 226. In some embodiments, the antisense strand comprises at least 21 contiguous nucleotides in any one of the nucleotide sequences of SEQ ID NO: 10 to SEQ ID NO: 18 and SEQ ID NO: 123 to SEQ ID NO: 226.

In some embodiments, the sense strand comprises or is selected from the group consisting of any one of the following nucleotide sequences: SEQ ID NO: 1 to SEQ ID NO: 9 and SEQ ID NO: 19 to SEQ ID NO: 122.

In some embodiments, the antisense strand comprises or is selected from the group consisting of any one of the following nucleotide sequences: SEQ ID NO: 10 to SEQ ID NO: 18 and SEQ ID NO: 123 to SEQ ID NO: 226.

In some embodiments, provided herein is an siRNA targeting inhibin βE, which comprises a sense strand and an antisense strand forming a double-stranded region, wherein the sense strand and the antisense strand comprise or are selected from the following groups:
1) a sense strand set forth in SEQ ID NO: 1 and an antisense strand set forth in SEQ ID NO: 10;
2) a sense strand set forth in SEQ ID NO: 2 and an antisense strand set forth in SEQ ID NO: 11;
3) a sense strand set forth in SEQ ID NO: 3 and an antisense strand set forth in SEQ ID NO: 12;
4) a sense strand set forth in SEQ ID NO: 4 and an antisense strand set forth in SEQ ID NO: 13;
5) a sense strand set forth in SEQ ID NO: 5 and an antisense strand set forth in SEQ ID NO: 14;
6) a sense strand set forth in SEQ ID NO: 6 and an antisense strand set forth in SEQ ID NO: 15;
7) a sense strand set forth in SEQ ID NO: 7 and an antisense strand set forth in SEQ ID NO: 16;
8) a sense strand set forth in SEQ ID NO: 8 and an antisense strand set forth in SEQ ID NO: 17;
9) a sense strand set forth in SEQ ID NO: 9 and an antisense strand set forth in SEQ ID NO: 18;
10) a sense strand set forth in SEQ ID NO: 19 and an antisense strand set forth in SEQ ID NO: 123;
11) a sense strand set forth in SEQ ID NO: 20 and an antisense strand set forth in SEQ ID NO: 124;
12) a sense strand set forth in SEQ ID NO: 21 and an antisense strand set forth in SEQ ID NO: 125;
13) a sense strand set forth in SEQ ID NO: 22 and an antisense strand set forth in SEQ ID NO: 126;
14) a sense strand set forth in SEQ ID NO: 23 and an antisense strand set forth in SEQ ID NO: 127;
15) a sense strand set forth in SEQ ID NO: 24 and an antisense strand set forth in SEQ ID NO: 128;
16) a sense strand set forth in SEQ ID NO: 25 and an antisense strand set forth in SEQ ID NO: 129;
17) a sense strand set forth in SEQ ID NO: 26 and an antisense strand set forth in SEQ ID NO: 130;
18) a sense strand set forth in SEQ ID NO: 27 and an antisense strand set forth in SEQ ID NO: 131;
19) a sense strand set forth in SEQ ID NO: 28 and an antisense strand set forth in SEQ ID NO: 132;
20) a sense strand set forth in SEQ ID NO: 29 and an antisense strand set forth in SEQ ID NO: 133;
21) a sense strand set forth in SEQ ID NO: 30 and an antisense strand set forth in SEQ ID NO: 134;
22) a sense strand set forth in SEQ ID NO: 31 and an antisense strand set forth in SEQ ID NO: 135;
23) a sense strand set forth in SEQ ID NO: 32 and an antisense strand set forth in SEQ ID NO: 136;
24) a sense strand set forth in SEQ ID NO: 33 and an antisense strand set forth in SEQ ID NO: 137;
25) a sense strand set forth in SEQ ID NO: 34 and an antisense strand set forth in SEQ ID NO: 138;
26) a sense strand set forth in SEQ ID NO: 35 and an antisense strand set forth in SEQ ID NO: 139;
27) a sense strand set forth in SEQ ID NO: 36 and an antisense strand set forth in SEQ ID NO: 140;
28) a sense strand set forth in SEQ ID NO: 37 and an antisense strand set forth in SEQ ID NO: 141;
29) a sense strand set forth in SEQ ID NO: 38 and an antisense strand set forth in SEQ ID NO: 142;
30) a sense strand set forth in SEQ ID NO: 39 and an antisense strand set forth in SEQ ID NO: 143;
31) a sense strand set forth in SEQ ID NO: 40 and an antisense strand set forth in SEQ ID NO: 144;
32) a sense strand set forth in SEQ ID NO: 41 and an antisense strand set forth in SEQ ID NO: 145;
33) a sense strand set forth in SEQ ID NO: 42 and an antisense strand set forth in SEQ ID NO: 146;
34) a sense strand set forth in SEQ ID NO: 43 and an antisense strand set forth in SEQ ID NO: 147;
35) a sense strand set forth in SEQ ID NO: 44 and an antisense strand set forth in SEQ ID NO: 148;
36) a sense strand set forth in SEQ ID NO: 45 and an antisense strand set forth in SEQ ID NO: 149;
37) a sense strand set forth in SEQ ID NO: 46 and an antisense strand set forth in SEQ ID NO: 150;
38) a sense strand set forth in SEQ ID NO: 47 and an antisense strand set forth in SEQ ID NO: 151;
39) a sense strand set forth in SEQ ID NO: 48 and an antisense strand set forth in SEQ ID NO: 152;
40) a sense strand set forth in SEQ ID NO: 49 and an antisense strand set forth in SEQ ID NO: 153;
41) a sense strand set forth in SEQ ID NO: 50 and an antisense strand set forth in SEQ ID NO: 154;
42) a sense strand set forth in SEQ ID NO: 51 and an antisense strand set forth in SEQ ID NO: 155;
43) a sense strand set forth in SEQ ID NO: 52 and an antisense strand set forth in SEQ ID NO: 156;
44) a sense strand set forth in SEQ ID NO: 53 and an antisense strand set forth in SEQ ID NO: 157;
45) a sense strand set forth in SEQ ID NO: 54 and an antisense strand set forth in SEQ ID NO: 158;
46) a sense strand set forth in SEQ ID NO: 55 and an antisense strand set forth in SEQ ID NO: 159;
47) a sense strand set forth in SEQ ID NO: 56 and an antisense strand set forth in SEQ ID NO: 160;
48) a sense strand set forth in SEQ ID NO: 57 and an antisense strand set forth in SEQ ID NO: 161;
49) a sense strand set forth in SEQ ID NO: 58 and an antisense strand set forth in SEQ ID NO: 162;
50) a sense strand set forth in SEQ ID NO: 59 and an antisense strand set forth in SEQ ID NO: 163;
51) a sense strand set forth in SEQ ID NO: 60 and an antisense strand set forth in SEQ ID NO: 164;
52) a sense strand set forth in SEQ ID NO: 61 and an antisense strand set forth in SEQ ID NO: 165;
53) a sense strand set forth in SEQ ID NO: 62 and an antisense strand set forth in SEQ ID NO: 166;
54) a sense strand set forth in SEQ ID NO: 63 and an antisense strand set forth in SEQ ID NO: 167;
55) a sense strand set forth in SEQ ID NO: 64 and an antisense strand set forth in SEQ ID NO: 168;
56) a sense strand set forth in SEQ ID NO: 65 and an antisense strand set forth in SEQ ID NO: 169;
57) a sense strand set forth in SEQ ID NO: 66 and an antisense strand set forth in SEQ ID NO: 170;
58) a sense strand set forth in SEQ ID NO: 67 and an antisense strand set forth in SEQ ID NO: 171;
59) a sense strand set forth in SEQ ID NO: 68 and an antisense strand set forth in SEQ ID NO: 172;
60) a sense strand set forth in SEQ ID NO: 69 and an antisense strand set forth in SEQ ID NO: 173;
61) a sense strand set forth in SEQ ID NO: 70 and an antisense strand set forth in SEQ ID NO: 174;
62) a sense strand set forth in SEQ ID NO: 71 and an antisense strand set forth in SEQ ID NO: 175;
63) a sense strand set forth in SEQ ID NO: 72 and an antisense strand set forth in SEQ ID NO: 176;
64) a sense strand set forth in SEQ ID NO: 73 and an antisense strand set forth in SEQ ID NO: 177;
65) a sense strand set forth in SEQ ID NO: 74 and an antisense strand set forth in SEQ ID NO: 178;
66) a sense strand set forth in SEQ ID NO: 75 and an antisense strand set forth in SEQ ID NO: 179;
67) a sense strand set forth in SEQ ID NO: 76 and an antisense strand set forth in SEQ ID NO: 180;
68) a sense strand set forth in SEQ ID NO: 77 and an antisense strand set forth in SEQ ID NO: 181;
69) a sense strand set forth in SEQ ID NO: 78 and an antisense strand set forth in SEQ ID NO: 182;
70) a sense strand set forth in SEQ ID NO: 79 and an antisense strand set forth in SEQ ID NO: 183;
71) a sense strand set forth in SEQ ID NO: 80 and an antisense strand set forth in SEQ ID NO: 184;
72) a sense strand set forth in SEQ ID NO: 81 and an antisense strand set forth in SEQ ID NO: 185;
73) a sense strand set forth in SEQ ID NO: 82 and an antisense strand set forth in SEQ ID NO: 186;
74) a sense strand set forth in SEQ ID NO: 83 and an antisense strand set forth in SEQ ID NO: 187;
75) a sense strand set forth in SEQ ID NO: 84 and an antisense strand set forth in SEQ ID NO: 188;
76) a sense strand set forth in SEQ ID NO: 85 and an antisense strand set forth in SEQ ID NO: 189;
77) a sense strand set forth in SEQ ID NO: 86 and an antisense strand set forth in SEQ ID NO: 190;
78) a sense strand set forth in SEQ ID NO: 87 and an antisense strand set forth in SEQ ID NO: 191;
79) a sense strand set forth in SEQ ID NO: 88 and an antisense strand set forth in SEQ ID NO: 192;
80) a sense strand set forth in SEQ ID NO: 89 and an antisense strand set forth in SEQ ID NO: 193;
81) a sense strand set forth in SEQ ID NO: 90 and an antisense strand set forth in SEQ ID NO: 194;
82) a sense strand set forth in SEQ ID NO: 91 and an antisense strand set forth in SEQ ID NO: 195;
83) a sense strand set forth in SEQ ID NO: 92 and an antisense strand set forth in SEQ ID NO: 196;
84) a sense strand set forth in SEQ ID NO: 93 and an antisense strand set forth in SEQ ID NO: 197;
85) a sense strand set forth in SEQ ID NO: 94 and an antisense strand set forth in SEQ ID NO: 198;
86) a sense strand set forth in SEQ ID NO: 95 and an antisense strand set forth in SEQ ID NO: 199;
87) a sense strand set forth in SEQ ID NO: 96 and an antisense strand set forth in SEQ ID NO: 200;
88) a sense strand set forth in SEQ ID NO: 97 and an antisense strand set forth in SEQ ID NO: 201;
89) a sense strand set forth in SEQ ID NO: 98 and an antisense strand set forth in SEQ ID NO: 202;
90) a sense strand set forth in SEQ ID NO: 99 and an antisense strand set forth in SEQ ID NO: 203;
91) a sense strand set forth in SEQ ID NO: 100 and an antisense strand set forth in SEQ ID NO: 204;
92) a sense strand set forth in SEQ ID NO: 101 and an antisense strand set forth in SEQ ID NO: 205;
93) a sense strand set forth in SEQ ID NO: 102 and an antisense strand set forth in SEQ ID NO: 206;
94) a sense strand set forth in SEQ ID NO: 103 and an antisense strand set forth in SEQ ID NO: 207;
95) a sense strand set forth in SEQ ID NO: 104 and an antisense strand set forth in SEQ ID NO: 208;
96) a sense strand set forth in SEQ ID NO: 105 and an antisense strand set forth in SEQ ID NO: 209;
97) a sense strand set forth in SEQ ID NO: 106 and an antisense strand set forth in SEQ ID NO: 210;
98) a sense strand set forth in SEQ ID NO: 107 and an antisense strand set forth in SEQ ID NO: 211;
99) a sense strand set forth in SEQ ID NO: 108 and an antisense strand set forth in SEQ ID NO: 212;
100) a sense strand set forth in SEQ ID NO: 109 and an antisense strand set forth in SEQ ID NO: 213;
101) a sense strand set forth in SEQ ID NO: 110 and an antisense strand set forth in SEQ ID NO: 214;
102) a sense strand set forth in SEQ ID NO: 111 and an antisense strand set forth in SEQ ID NO: 215;
103) a sense strand set forth in SEQ ID NO: 112 and an antisense strand set forth in SEQ ID NO: 216;
104) a sense strand set forth in SEQ ID NO: 113 and an antisense strand set forth in SEQ ID NO: 217;
105) a sense strand set forth in SEQ ID NO: 114 and an antisense strand set forth in SEQ ID NO: 218;
106) a sense strand set forth in SEQ ID NO: 115 and an antisense strand set forth in SEQ ID NO: 219;
107) a sense strand set forth in SEQ ID NO: 116 and an antisense strand set forth in SEQ ID NO: 220;
108) a sense strand set forth in SEQ ID NO: 117 and an antisense strand set forth in SEQ ID NO: 221;
109) a sense strand set forth in SEQ ID NO: 118 and an antisense strand set forth in SEQ ID NO: 222;
110) a sense strand set forth in SEQ ID NO: 119 and an antisense strand set forth in SEQ ID NO: 223;
111) a sense strand set forth in SEQ ID NO: 120 and an antisense strand set forth in SEQ ID NO: 224;
112) a sense strand set forth in SEQ ID NO: 121 and an antisense strand set forth in SEQ ID NO: 225; and
113) a sense strand set forth in SEQ ID NO: 122 and an antisense strand set forth in SEQ ID NO: 226.

In some embodiments, at least one nucleotide in the sense strand and/or the antisense strand is a modified nucleotide.

In some embodiments, all of the nucleotides are modified nucleotides.

In some embodiments, the modified nucleotide is a 2'-methoxy-modified nucleotide or a 2'-fluoro-modified nucleotide.

In some embodiments, three contiguous nucleotides in the sense strand of the siRNA are 2'-fluoro-modified nucleotides.

In some embodiments, three contiguous nucleotides at positions 7-9 of the 5' end in the sense strand of the siRNA are 2'-fluoro-modified nucleotides.

In some embodiments, three contiguous nucleotides at positions 7-9 of the 5' end in the sense strand of the siRNA are 2'-fluoro-modified nucleotides, and the remaining positions of the sense strand are all 2'-methoxy-modified nucleotides. In some embodiments, four nucleotides at position 5 and positions 7-9 of the 5' end in the sense strand are each independently a 2'-fluoro-modified nucleotide, and the remaining positions of the sense strand are all 2'-methoxy-modified nucleotides.

In some embodiments, in the direction from the 5' end to the 3' end, the nucleotides at positions 2, 6, 12, 14, and 16 of the antisense strand are each independently a 2'-fluoro-modified nucleotide, and the nucleotides at the remaining positions are 2'-methoxy-modified nucleotides. In some embodiments, in the direction from the 5' end to the 3' end, the nucleotides at positions 2, 4, 6, 10, 12, 14, 16, and 18 of the antisense strand are each independently a 2'-fluoro-modified nucleotide, and the nucleotides at the remaining positions are 2'-methoxy-modified nucleotides. In some embodiments, in the direction from the 5' end to the 3' end, the nucleotides at positions 2, 6, 14, and 16 of the antisense strand are each independently a 2'-fluoro-modified nucleotide, and the nucleotides at the remaining positions are 2'-methoxy-modified nucleotides.

In some embodiments, at least one phosphodiester group in the sense strand and/or the antisense strand is a phosphodiester group with a modification group. The modification group endows the siRNA with increased stability in a biological sample or environment. In some embodiments, the phosphodiester group with a modification group is a phosphorothioate diester group.

In some embodiments, the phosphorothioate diester group is present in at least one of the following positions selected from the group consisting of:
a position between the 1st and 2nd nucleotides at the 5' end of the sense strand;
a position between the 2nd and 3rd nucleotides at the 5' end of the sense strand;
a position between the 1st and 2nd nucleotides at the 3' end of the sense strand;
a position between the 2nd and 3rd nucleotides at the 3' end of the sense strand;
a position between the 1st and 2nd nucleotides at the 5' end of the antisense strand;
a position between the 2nd and 3rd nucleotides at the 5' end of the antisense strand;
a position between the 1st and 2nd nucleotides at the 3' end of the antisense strand; and
a position between the 2nd and 3rd nucleotides at the 3' end of the antisense strand.

In some embodiments, the sense strand and/or the antisense strand comprise a plurality of phosphorothioate diester groups, and the phosphorothioate diester groups are present:
between the 1st and 2nd nucleotides at the 5' end of the sense strand; and
between the 2nd and 3rd nucleotides at the 5' end of the sense strand; and
between the 1st and 2nd nucleotides at the 5' end of the antisense strand; and
between the 2nd and 3rd nucleotides at the 5' end of the antisense strand; and
between the 1st and 2nd nucleotides at the 3' end of the antisense strand; and
between the 2nd and 3rd nucleotides at the 3' end of the antisense strand.

The present disclosure also provides an siRNA conjugate, which comprises any one of the siRNAs described above and a targeting ligand linked to an end of the siRNA.

In some embodiments, the siRNA and the targeting ligand are linked covalently or non-covalently.

In some embodiments, the targeting ligand targets the liver; in some embodiments, the targeting ligand binds to an asialoglycoprotein receptor (ASGPR); in some embodiments, the targeting ligand comprises a galactose cluster or a galactose derivative cluster, wherein the galactose derivative is selected from the group consisting of N-acetyl-galactosamine, N-trifluoroacetylgalactosamine, N-propionylgalactosamine, N-n-butyrylgalactosamine, and N-isobutyrylgalactosamine.

In some embodiments, the targeting ligand comprises one or more of N-acetyl-galactosamine, N-trifluoroacetylgalactosamine, N-propionylgalactosamine, N-n-butyrylgalactosamine, or N-isobutyrylgalactosamine; preferably, the targeting ligand comprises N-acetyl-galactosamine. In some embodiments, the targeting ligand is linked to the 3' end of the sense strand of the siRNA.

In some embodiments, the targeting ligand is linked to the 3' end or the 5' end of the sense strand or the antisense strand in the siRNA via a phosphodiester group, a phosphorothioate diester group, or a phosphonic acid group.

In some embodiments, the targeting ligand is linked to an end of the siRNA via a phosphodiester group.

In some embodiments, the targeting ligand is indirectly linked to an end of the siRNA via a phosphodiester group, a phosphorothioate diester group, or a phosphonic acid group. In some embodiments, the targeting ligand is indirectly linked to an end of the siRNA via a phosphodiester group.

In some embodiments, the targeting ligand is directly linked to an end of the siRNA via a phosphodiester group, a phosphorothioate diester group, or a phosphonic acid group; in some embodiments, the targeting ligand is directly linked to an end of the siRNA via a phosphodiester group.

In some embodiments, the targeting ligand is directly linked to the 3' end of the sense strand of the siRNA via a phosphodiester group or a phosphorothioate diester group; in some embodiments, the targeting ligand is directly linked to the 3' end of the sense strand of the siRNA via a phosphodiester group.

In some embodiments, the targeting moiety of the targeting ligand consists of one or more targeting groups or targeting moieties, and the targeting ligand assists in directing the delivery of a therapeutic agent linked thereto to the desired target location. In some cases, the targeting moiety may bind to a cell or cellular receptor and initiate endocytosis to promote entry of the therapeutic agent into the cell. The targeting moiety may comprise a compound with affinity for a cellular receptor or a cell surface molecule or an antibody. Various targeting ligands comprising targeting moieties may be linked to therapeutic agents and other compounds to target the agents at cells and specific cellular receptors.

In some embodiments, the types of the targeting moieties include carbohydrates, cholesterol and cholesterol groups, or steroids. Targeting moieties capable of binding to cellular receptors include saccharides such as galactose, galactose derivatives (e.g., N-acetyl-galactosamine, N-trifluoroacetylgalactosamine, N-propionylgalactosamine, N-n-butyrylgalactosamine, and N-isobutyrylgalactosamine), mannose, and mannose derivatives).

Targeting moieties that bind to asialoglycoprotein receptors (ASGPRs) are known to be particularly used for directing the delivery of oligomeric compounds to the liver. Asialoglycoprotein receptors are extensively expressed on liver cells (hepatocytes). The targeting moieties of cellular receptors targeting ASGPR include galactose and galactose derivatives. Specifically, clusters of galactose derivatives, including clusters consisting of 2, 3, 4, or more than 4 N-acetyl-galactosamine (GalNAc or NAG), can promote the uptake of certain compounds in hepatocytes. The GalNAc cluster coupled to the oligomeric compound is used for directing the composition to the liver where the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into the interior of the cell.

In some embodiments, the targeting ligand may comprise 2, 3, 4, or more than 4 targeting moieties. In some embodiments, the targeting ligand disclosed herein may comprise 1, 2, 3, 4, or more than 4 targeting moieties linked to a branching group via L₂.

In some embodiments, each targeting moiety comprises a galactosamine derivative, which is N-acetyl-galactosamine. Other sugars that may be used as targeting moieties and that have affinity for asialoglycoprotein receptors may be selected from the group consisting of galactose, galactosamine, N-formyl-galactosamine, N-acetyl-galactosamine, N-propionyl-galactosamine, N-n-butyryl-galactosamine, N-isobutyrylgalactosamine, etc.

In some embodiments, the targeting ligand in the present disclosure comprises N-acetyl-galactosamine as a targeting moiety,

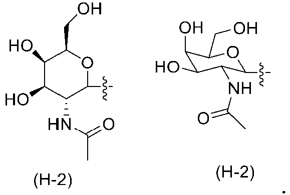

In some embodiments, the targeting ligand comprises three terminal galactosamines or galactosamine derivatives (such as N-acetyl-galactosamine), each of which has affinity for asialoglycoprotein receptors. In some embodiments, the targeting ligand comprises three terminal N-acetyl-galactosamines (GalNAc or NAG) as targeting moieties.

In some embodiments, the targeting ligand comprises four terminal galactosamines or galactosamine derivatives (such as N-acetyl-galactosamine), each of which has affinity for asialoglycoprotein receptors. In some embodiments, the targeting ligand comprises four terminal N-acetyl-galactosamines (GalNAc or NAG) as targeting moieties.

The terms commonly used in the art when referring to three terminal N-acetyl-galactosamines include
tri-antennary, tri-valent, and trimer.

The terms commonly used in the art when referring to four terminal N-acetyl-galactosamines include
tetra-antennary, tetra-valent, and tetramer.

In some embodiments, the targeting ligand provided by the present disclosure is a compound represented by formula (II) or formula (III) or a pharmaceutically acceptable salt thereof,

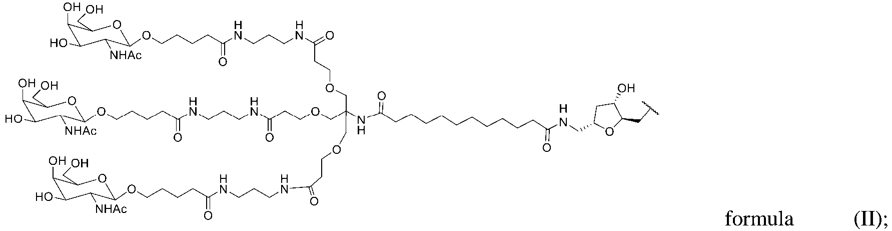

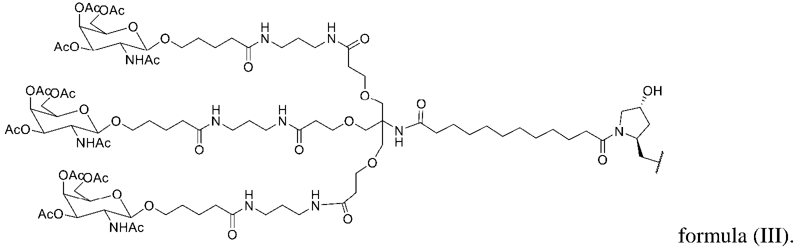

In some embodiments, the N-acetyl-galactosamine moiety in the above targeting ligands may be replaced with N-trifluoroacetylgalactosamine, N-propionylgalactosamine, N-n-butyrylgalactosamine, or N-isobutyrylgalactosamine.

In some embodiments, the siRNA and/or the siRNA conjugate of the present disclosure have nucleotide sequences set forth in any one of the sense and antisense strand pairs in Tables 1a and 1b or Tables 2a and 2b.

In some embodiments, the siRNA and/or the siRNA conjugate of the present disclosure comprises or is selected from the group consisting of any one of SEQ ID NO: 275 to SEQ ID NO: 383.

In some embodiments, the sense strand in the siRNA and/or the siRNA conjugate of the present disclosure comprises or is selected from the group consisting of the nucleotide sequence set forth in any one of SEQ ID NO: 227 to SEQ ID NO: 242, SEQ ID NO: 243 to SEQ ID NO: 250, and SEQ ID NO: 275 to SEQ ID NO: 383; and/or the antisense strand comprises or is selected from the group consisting of the nucleotide sequence set forth in any one of SEQ ID NO: 251 to SEQ ID NO: 266, SEQ ID NO: 267 to SEQ ID NO: 274, and SEQ ID NO: 384 to SEQ ID NO: 492.

In some embodiments, the sense strand and the antisense strand comprise or are selected from the following groups: 1) a sense strand set forth in SEQ ID NO: 227 and an antisense strand set forth in SEQ ID NO: 251;
2) a sense strand set forth in SEQ ID NO: 228 and an antisense strand set forth in SEQ ID NO: 252;
3) a sense strand set forth in SEQ ID NO: 229 and an antisense strand set forth in SEQ ID NO: 253;
4) a sense strand set forth in SEQ ID NO: 230 and an antisense strand set forth in SEQ ID NO: 254;
5) a sense strand set forth in SEQ ID NO: 231 and an antisense strand set forth in SEQ ID NO: 255;
6) a sense strand set forth in SEQ ID NO: 232 and an antisense strand set forth in SEQ ID NO: 256;
7) a sense strand set forth in SEQ ID NO: 233 and an antisense strand set forth in SEQ ID NO: 257;
8) a sense strand set forth in SEQ ID NO: 234 and an antisense strand set forth in SEQ ID NO: 258;
9) a sense strand set forth in SEQ ID NO: 235 and an antisense strand set forth in SEQ ID NO: 259;
10) a sense strand set forth in SEQ ID NO: 236 and an antisense strand set forth in SEQ ID NO: 260;
11) a sense strand set forth in SEQ ID NO: 237 and an antisense strand set forth in SEQ ID NO: 261;
12) a sense strand set forth in SEQ ID NO: 238 and an antisense strand set forth in SEQ ID NO: 262;
13) a sense strand set forth in SEQ ID NO: 239 and an antisense strand set forth in SEQ ID NO: 263;
14) a sense strand set forth in SEQ ID NO: 240 and an antisense strand set forth in SEQ ID NO: 264;
15) a sense strand set forth in SEQ ID NO: 241 and an antisense strand set forth in SEQ ID NO: 265;
16) a sense strand set forth in SEQ ID NO: 242 and an antisense strand set forth in SEQ ID NO: 266;
17) a sense strand set forth in SEQ ID NO: 243 and an antisense strand set forth in SEQ ID NO: 267;
18) a sense strand set forth in SEQ ID NO: 244 and an antisense strand set forth in SEQ ID NO: 268;
19) a sense strand set forth in SEQ ID NO: 245 and an antisense strand set forth in SEQ ID NO: 269;
20) a sense strand set forth in SEQ ID NO: 246 and an antisense strand set forth in SEQ ID NO: 270;
21) a sense strand set forth in SEQ ID NO: 247 and an antisense strand set forth in SEQ ID NO: 271;
22) a sense strand set forth in SEQ ID NO: 248 and an antisense strand set forth in SEQ ID NO: 272;
23) a sense strand set forth in SEQ ID NO: 249 and an antisense strand set forth in SEQ ID NO: 273; and
24) a sense strand set forth in SEQ ID NO: 250 and an antisense strand set forth in SEQ ID NO: 274.

In another aspect, the present disclosure provides a composition, which comprises the siRNA and the siRNA conjugate described in the present disclosure, and one or more pharmaceutically acceptable excipients, such as vehicles, carriers, diluents, and/or delivery polymers.

Various delivery systems are known and can be used for the siRNA or the siRNA conjugate of the present disclosure, e.g., encapsulation in liposomes, microparticles, microcapsules, recombinant cells capable of expressing the siRNA or the siRNA conjugate, receptor-mediated endocytosis, and construction of a nucleic acid as part of retroviral or other vectors.

In another aspect, the present disclosure provides use of the siRNA, the siRNA conjugate, or the composition described above in the manufacture of a medicament for treating a disease in a subject; in some embodiments, the disease is selected from the group consisting of a hepatic disease.

In another aspect, the present disclosure provides a method for treating a disease in a subject, which comprises administering to the subject the siRNA, the siRNA conjugate, or the composition described above.

In another aspect, the present disclosure provides a method for inhibiting mRNA expression in a subject, which comprises administering to the subject the siRNA, the siRNA conjugate, or the composition described above.

In another aspect, the present disclosure provides a method for delivering an expression-inhibiting oligomeric compound to the liver *in vivo,* which comprises administering to a subject the siRNA, the siRNA conjugate, or the composition described above.

The siRNA, the siRNA conjugate, or the composition, and the methods disclosed herein can reduce the level of a target mRNA in a cell, a cell population, a cell population, a tissue, or a subject, which comprises: administering to the subject a therapeutically effective amount of the siRNA, the siRNA conjugate, or the composition described in the present disclosure. The siRNA is linked to a targeting ligand, thereby inhibiting the expression of the target mRNA in the subject.

In some embodiments, the subject has been previously identified as having pathological up-regulation of the target gene in the targeted cell or tissue.

The subject described in the present disclosure refers to a subject having (or suspected to have or susceptible to) a disease or disorder that would benefit from reduction or inhibition of target mRNA expression.

Delivery can be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In optional embodiments, the pharmaceutical composition provided by the present disclosure may be administered by injection, e.g., intravenous, intramuscular, intradermal, subcutaneous, intraduodenal, or intraperitoneal injection.

In optional embodiments, after the targeting ligand and the siRNA are linked to form a conjugate, the conjugate can be packaged in a kit.

In another aspect, the present disclosure also provides a pharmaceutical composition, which comprises the siRNA or the siRNA conjugate of the present disclosure.

In some embodiments, the pharmaceutical composition may further comprise a pharmaceutically acceptable auxiliary material and/or adjuvant, and the auxiliary material may be one or more of various formulations or compounds conventionally used in the art. For example, the pharmaceutically acceptable auxiliary material may include at least one of a pH buffer, a protective agent, and an osmotic pressure regulator.

In some embodiments, a unit dose of the pharmaceutical composition is 0.001 mg to 1000 mg.

In certain embodiments, the pharmaceutical composition comprises 0.01% to 99.99% of the aforementioned compound or a pharmaceutically acceptable salt thereof or an isotopically substituted form thereof based on the total weight of the composition. In certain embodiments, the pharmaceutical composition comprises 0.1% to 99.9% of the aforementioned compound or the pharmaceutically acceptable salt thereof or the isotopically substituted form thereof. In certain embodiments, the pharmaceutical composition comprises 0.5% to 99.5% of the aforementioned compound or the pharmaceutically acceptable salt thereof or the isotopically substituted form thereof. In certain embodiments, the pharmaceutical composition comprises 1% to 99% of the aforementioned compound or the pharmaceutically acceptable salt thereof or the isotopically substituted form thereof. In certain embodiments, the pharmaceutical composition comprises 2% to 98% of the aforementioned compound or the pharmaceutically acceptable salt thereof or the isotopically substituted form thereof.

In certain embodiments, the pharmaceutical composition comprises 0.01% to 99.99% of a pharmaceutically acceptable excipient based on the total weight of the composition. In certain embodiments, the pharmaceutical composition comprises 0.1% to 99.9% of the pharmaceutically acceptable excipient. In certain embodiments, the pharmaceutical composition comprises 0.5% to 99.5% of the pharmaceutically acceptable excipient. In certain embodiments, the pharmaceutical composition comprises 1% to 99% of the pharmaceutically acceptable excipient. In certain embodiments, the pharmaceutical composition comprises 2% to 98% of the pharmaceutically acceptable excipient.

In some embodiments, when the siRNA, the siRNA conjugate, or the pharmaceutical composition described above is in contact with a target gene-expressing cell, the siRNA conjugate or the pharmaceutical composition described above inhibits the target gene expression by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate or the pharmaceutical composition described above results in a percent remaining expression of the target gene mRNA of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

The present disclosure also provides a cell, which comprises the siRNA or the siRNA conjugate of the present disclosure.

The present disclosure also provides a kit, which comprises the siRNA, the siRNA conjugate, or the pharmaceutical composition of the present disclosure.

The present disclosure also provides a method for silencing a target gene or mRNA of a target gene in a cell, which comprises the step of introducing into the cell the siRNA, the siRNA conjugate, and/or the pharmaceutical composition according to the present disclosure.

The present disclosure also provides a method for silencing a target gene or mRNA of a target gene in a cell *in vivo* or *in vitro,* which comprises the step of introducing into the cell the siRNA, the siRNA conjugate, and/or the pharmaceutical composition according to the present disclosure.

The present disclosure also provides a method for inhibiting the expression of a target gene or mRNA of a target gene, which comprises administering to a subject in need thereof an effective amount or an effective dose of the siRNA, the siRNA conjugate, and/or the pharmaceutical composition according to the present disclosure.

In some embodiments, administration is performed through administration modes including intramuscular, intrabronchial, intrapleural, intraperitoneal, intraarterial, lymphatic, intravenous, subcutaneous, cerebrospinal, or combinations thereof.

In some embodiments, the effective amount or the effective dose of the siRNA, the siRNA conjugate, and/or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

In some embodiments, the target gene is inhibin βE (INHBE).

The present disclosure provides the aforementioned siRNA and/or pharmaceutical composition and/or siRNA conjugate for use in treating and/or preventing a disease associated with inhibin βE (INHBE) gene expression in a subject; in some embodiments, the disease is selected from the group consisting of metabolic disorders; in some embodiments, the disease is selected from the group consisting of metabolic syndrome (MS), a cardiovascular disease, obesity, hepatitis, and a renal disease.

The present disclosure provides a method for treating and/or preventing a disease associated with inhibin βE (INHBE) gene expression in a subject, which comprises administering to the subject an effective amount or an effective dose of the siRNA, the siRNA conjugate, or the pharmaceutical composition described in the present disclosure; in some embodiments, the disease is selected from the group consisting of metabolic disorders; in some embodiments, the disease is selected from the group consisting of metabolic syndrome (MS), a cardiovascular disease, obesity, hepatitis, and a renal disease.

The present disclosure provides use of the aforementioned siRNA and/or pharmaceutical composition and/or siRNA conjugate in the manufacture of a medicament for treating and/or preventing a disease associated with inhibin βE (INHBE) gene expression; in some embodiments, the disease is selected from the group consisting of metabolic disorders; in some embodiments, the disease is selected from the group consisting of metabolic syndrome (MS), a cardiovascular disease, obesity, hepatitis, and a renal disease.

The present disclosure provides use of the aforementioned siRNA and/or pharmaceutical composition and/or siRNA conjugate in the manufacture of a medicament for inhibiting the expression of INHBE.

The present disclosure provides a method for inhibiting the expression of INHBE, which comprises administering to a subject an effective amount or an effective dose of the aforementioned siRNA and/or pharmaceutical composition and/or siRNA conjugate.

The present disclosure provides the aforementioned siRNA and/or pharmaceutical composition and/or siRNA conjugate for use in treating and/or preventing a disease; in some embodiments, the disease is selected from the group consisting of metabolic disorders; in some embodiments, the disease is selected from the group consisting of metabolic syndrome (MS), a cardiovascular disease, obesity, hepatitis, and a renal disease.

The present disclosure provides a method for treating and/or preventing a disease, which comprises administering to a subject an effective amount or an effective dose of the aforementioned siRNA and/or pharmaceutical composition and/or siRNA conjugate; in some embodiments, the disease is selected from the group consisting of metabolic disorders; in some embodiments, the disease is selected from the group consisting of metabolic syndrome (MS), a cardiovascular disease, obesity, hepatitis, and a renal disease.

The present disclosure provides use of the aforementioned siRNA and/or pharmaceutical composition and/or siRNA conjugate in the manufacture of a medicament for treating and/or preventing a disease; in some embodiments, the disease is selected from the group consisting of metabolic disorders; in some embodiments, the disease is selected from the group consisting of metabolic syndrome (MS), a cardiovascular disease, obesity, hepatitis, and a renal disease.

The present disclosure provides a method for delivering an siRNA that inhibits the expression and/or replication of INHBE to the liver *in vivo,* which comprises administering to a subject the aforementioned siRNA and/or pharmaceutical composition and/or siRNA conjugate.

The present disclosure also provides a method for preparing an siRNA or an siRNA conjugate, which comprises synthesizing the siRNA or the siRNA conjugate described in the present disclosure.

The present disclosure also provides an siRNA or an siRNA conjugate, wherein one or more bases U, e.g., 1, 2, 3, 3, 5, 6, 7, 8, 9, 10, or all bases U, of any one of the siRNAs or siRNA conjugates of the present disclosure are replaced with bases T.

The pharmaceutically acceptable salts of the compounds described in the present disclosure are selected from the group consisting of inorganic salts and organic salts. The compounds described in the present disclosure can react with acidic or basic substances to form corresponding salts.

In another aspect, where the configuration is not specified, the compounds of the present disclosure may be present in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure.

In addition, where the configuration is not specified, the compounds and intermediates of the present disclosure may also be present in different tautomeric forms, and all such forms are included within the scope of the present disclosure. The term "tautomer" or "tautomeric form" refers to structural isomers of different energies that can interconvert via a low-energy barrier.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-enantiomers and D- and L-isomers can be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. Furthermore, separation of enantiomers and diastereomers is generally accomplished by chromatography using a chiral stationary phase, optionally in combination with chemical derivatization (e.g., carbamate formation from amines).

The present disclosure also includes some isotopically labeled compounds of the present disclosure that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure further includes various deuterated forms of the compounds of formula I and formula II. Each available hydrogen atom linked to a carbon atom may be independently replaced by a deuterium atom. Those skilled in the art are able to synthesize the deuterated forms of the compounds of formula I and formula II with reference to the relevant literature. Commercially available deuterated starting materials can be used in preparing the deuterated forms of the compounds of formula I and formula II, or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

Where the configurations are not specified, in the chemical structures of the compounds of the present disclosure, a bond " 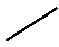 " indicates an unspecified configuration; that is, if chiral isomers exist in the chemical structures, the bond " 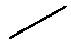 " may be " 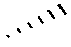 " or " 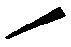 ", or includes both the configurations " 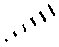 " and " 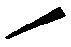 ". Although all of the above structural formulas are drawn as certain isomeric forms for the sake of simplicity, the present disclosure may include all isomers, such as tautomers, rotamers, geometric isomers, diastereomers, racemates, and enantiomers. In the chemical structures of the compounds of the present disclosure, a bond " 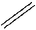 " does not specify a configuration; that is, the configuration for the bond " 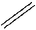 " can be an E configuration or a Z configuration, or includes both the E configuration and the Z configuration.

### Terms and Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

Unless otherwise specified, in the context of the present disclosure, the terms "inhibin βE" and "INHBE" are used interchangeably in the present disclosure. INHBEs include, but are not limited to, human INHBE, cynomolgus monkey INHBE, mouse INHBE, and rat INHBE, the amino acids and complete coding sequences and mRNA sequences of which are readily accessible using publicly available databases, e.g., GenBank, UniProt, OMIM, and the Macaca genome project website.

The term "INHBE" also refers to naturally occurring DNA sequence variations of the INHBE gene, such as a single nucleotide polymorphism (SNP) in the INHBE gene. Exemplary SNPs may be found in the dbSNP database.

The term "target sequence" refers to a contiguous portion of the nucleotide sequence of an mRNA molecule formed during the transcription of INHBE, including an mRNA that is a product of RNA processing of a primary transcription product. The targeted portion in the target sequence should be long enough to serve as a substrate for iRNA-directed cleavage. In one embodiment, the target sequence is within the protein encoding region of INHBE.

As used herein, in the context of RNA-mediated gene silencing, the sense strand (also referred to as SS or SS strand) refers to a strand that comprises a sequence that is identical or substantially identical to a target mRNA sequence; the antisense strand (also referred to as AS or AS strand) refers to a strand having a sequence complementary to a target mRNA sequence.

In the context describing the siRNA sense strand described herein, the term "a sequence differing from any one of the nucleotide sequences of SEQ ID NO: 1 to SEQ ID NO: 106 by no more than 3 nucleotides and comprises at least 15 contiguous nucleotides" is intended to mean that the siRNA sense strand described herein comprises at least 15 contiguous nucleotides of any one of the sense strands in SEQ ID NO: 1 to SEQ ID NO: 106 or differs from at least 15 contiguous nucleotides of any one of the sense strands in SEQ ID NO: 1 to SEQ ID NO: 106 by no more than 3 nucleotides (optionally, by no more than 2 nucleotides; optionally, by 1 nucleotide).

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably.

Unless otherwise specified, in the context of the present disclosure, "G", "C", "A", "T", and "U" each represent a nucleotide and contain the bases guanine, cytosine, adenine, thymidine, and uracil, respectively. It is well-known to those skilled in the art that the mutual replacement between bases T and U does not significantly affect the properties of the RNAi agent sequence. In the sequences of the present disclosure, U can be arbitrarily replaced with T, and the sequences after the replacement are also within the claimed scope of the present disclosure. In the sequences of the present disclosure, for the same nucleic acid strand, by designating the direction from the 5' end to the 3' end as the direction from the left to the right, the lowercase letter m indicates that a nucleoside adjacent to the left of the letter m is a 2'-methoxy-modified nucleoside; the lowercase letter f indicates that a nucleoside adjacent to the left of the letter f is a 2'-fluoro-modified nucleoside; the lowercase letter s indicates that two nucleosides adjacent to the letter s are linked via a phosphorothioate diester group. Unless otherwise specified, the "RNAi agent", "nucleotide", "compound", "chemical modification", "oligonucleotide", "double-stranded RNAi inhibitor molecule", "siRNA", "siRNA conjugate", "dsRNA", "nucleic acid", and "RNAi" of the present disclosure can each independently be present in the form of a salt, mixed salts, or a non-salt (e.g., a free acid or free base). When being present in the form of a salt or mixed salts, it may be a pharmaceutically acceptable salt. The term "pharmaceutically acceptable salt" includes pharmaceutically acceptable acid addition salts and pharmaceutically acceptable base addition salts. When it is present in the form of a salt, part of the groups may be ionized to form anions/cations; for example, phosphodiester groups and phosphorothioate diester groups may be present in the form of anions, and the structures in the form of a salt corresponding to the following structures are also within the claimed scope of the present disclosure. Unless otherwise specified, the 3' position of the first nucleotide at the 3' end of each strand is hydroxy; the 5' position of the first nucleotide at the 5' end of each strand is hydroxy.

The modifications and linking groups described above separately have the structures shown in the table below, where Base represents a base:

| Structure | Name |
|---|---|
| 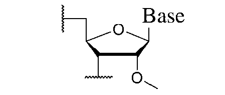 | 2'-Methoxy-modified nucleoside |
| 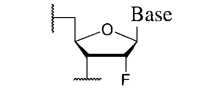 | 2'-Fluoro-modified nucleoside |
| 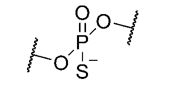 | Anion form of phosphorothioate diester group |
| 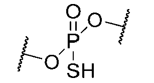 | Phosphorothioate diester group |
| 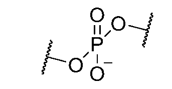 | Anion form of phosphodiester group |
| 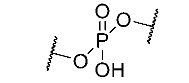 | Phosphodiester group |

As used in the present disclosure, the term "2'-fluoro-modified nucleotide" refers to a nucleotide in which the hydroxy group at the 2' position of the ribosyl group of the nucleotide is substituted with fluorine, and the term "non-2'-fluoro-modified nucleotide" refers to a nucleotide or a nucleotide analog in which the hydroxy group at the 2' position of the ribosyl group of the nucleotide is substituted with a non-fluorine group.

As used in the present disclosure, the term "2'-methoxy-modified nucleotide" refers to a nucleotide in which the 2'-hydroxy group of the ribosyl group is substituted with a methoxy group.

As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from an untreated or control (e.g., buffer-only control or inert agent control) treated subject, cell, or sample. For example, the remaining mRNA expression level can be used to characterize the degree of inhibition of target gene expression by the siRNA; for example, the remaining mRNA expression level is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using Dual-Glo^{®} Luciferase Assay System: the Firefly chemiluminescence value (Fir) and the Renilla chemiluminescence value (Ren) are each read, and the relative value Ratio = Ren/Fir is calculated; in the present disclosure, the ratio of the remaining mRNA expression level (or residual activity%) = Ratio (siRNA-treated group)/Ratio (siRNA-free control group), and the inhibition rate (%) = 100% - remaining mRNA expression level (%).

Unless otherwise specified, the "compound", "ligand", "nucleic acid-ligand conjugate", "siRNA conjugate", "nucleic acid", "conjugate", "chemical modification", "targeting ligand", "dsRNA", and "RNAi" of the present disclosure can each independently be present in the form of a salt, mixed salts, or a non-salt (e.g., a free acid or free base). When being present in the form of a salt or mixed salts, it may be a pharmaceutically acceptable salt.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts; sodium salts are preferred. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

"Effective amount" or "effective dose" refers to the amount of a drug, a compound, or a pharmaceutical composition necessary to obtain any one or more beneficial or desired therapeutic results. For prophylactic use, the beneficial or desired results include elimination or reduction of risk, reduction of severity or delay of the onset of a disorder, including the biochemistry, histology, and/or behavioral symptoms of the disorder, complications thereof, and intermediate pathological phenotypes that appear during the progression of the disorder. For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients.

As used herein, "patient", "subject", and "individual" are used interchangeably and include human or non-human animals, e.g., mammals, e.g., humans or monkeys.

The siRNA provided by the present disclosure may be obtained using a preparation method conventional in the art (e.g., solid-phase synthesis and liquid-phase synthesis). Solid-phase synthesis has been commercially available as a customization service. A modified nucleotide group can be introduced into the siRNA described in the present disclosure using a nucleoside monomer with a corresponding modification. Methods for preparing the nucleoside monomer with the corresponding modification and introducing the modified nucleotide group into the siRNA are also well-known to those skilled in the art.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs.

The terms "blunt end" and "blunt ended" are used interchangeably and mean that there are no unpaired nucleotides or nucleotide analogs at a given terminus of an siRNA, i.e., no nucleotide overhang. In most cases, an siRNA both ends of which are blunt ended will be double-stranded over its entire length.

The terms "about" and "approximately" mean that a numerical value is within an acceptable error range for the specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprise" should be assumed to be within an acceptable error range for that specific value.

Unless otherwise specified, "optionally" or "optional" means that the event or circumstance subsequently described may, but does not necessarily, occur, and this description includes instances where the event or circumstance occurs or does not occur. For example, "optionally, R₁ and R₂ are directly linked to form a ring" means that R₁ and R₂ being directly linked to form a ring may occur but does not necessarily exist, and this description includes an instance where R₁ and R₂ are directly linked to form a ring and an instance where R₁ and R₂ do not form a ring.

In the chemical structural formulas of the present disclosure, " 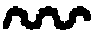 " or 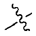 may be linked to any group or groups in accordance with the scope of the invention described herein.

In the context of the present disclosure, the 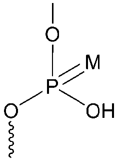 in the group 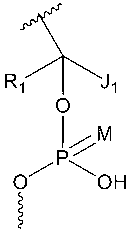 can be replaced with any group capable of linking to an adjacent nucleotide.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "direct linkage" means that a first compound or group is linked to a second compound or group without any atom or group of atoms interposed between.

The term "indirect linkage" means that a first compound or group is linked to a second compound or group via an intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, ketone, alkoxycarbonyl, aryloxycarbonyl, heteroaryloxycarbonyl, or halogen (e.g., F, Cl, Br, or I). When the substituent is a ketone or oxo (i.e., =O), two (2) hydrogens on the atom are replaced.

"Substituted with one or more..." refers to substitution with a single substituent or multiple substituents. In the case of substitution with multiple substituents, there may be a plurality of identical substituents, or one or a combination of a plurality of different substituents.

### DETAILED DESCRIPTION

The present disclosure is further described below with reference to examples, but these examples are not intended to limit the scope of the present disclosure. Experimental procedures without specific conditions specified in the examples of the present disclosure were generally conducted under conventional conditions such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated were commercially available conventional reagents.

### Example 1. Design of Human INHBE siRNAs

Using the human INHBE gene (NM_031479.5) as a target gene, siRNAs of 19/21 nt were designed in accordance with the general rules for active siRNAs. The sequences of the unmodified sense strands and antisense strands are shown in Tables 1a and 1b, and the sequences of the sense strands and antisense strands modified by 2'-fluoro, 2'-methoxy, etc. are detailed in Tables 2a and 2b.

**Table 1a. Unmodified sense strands and antisense strands**

| Double strand No. | SEQ ID NO: | SS strand (5'-3') | SEQ ID NO: | AS strand (5'-3') |
|---|---|---|---|---|
| TJR102661 | 1 | UCAAAGCUAUUUUCAUAAU | 10 | AUUAUGAAAAUAGCUUUGACU |
| TJR102664 | 2 | UAAUAAUACUAACAUGUUA | 11 | UAACAUGUUAGUAUUAUUAUG |
| TJR102668 | 3 | UUAAUAAAAAGGAGAAAGA | 12 | UCUUUCUCCUUUUUAUUAAGA |
| TJR102673 | 4 | AAAAGGAGAAAGAAAAUCA | 13 | UGAUUUUCUUUCUCCUUUUUA |
| TJR102667 | 5 | CAUAAUAAUACUAACAUGUUA | 14 | UAACAUGUUAGUAUUAUUAUGAA |
| TJR102826 | 6 | UAAUGGCAAUGUGGUCAAA | 15 | UUUGACCACAUUGCCAUUAUG |
| TJR102835 | 7 | AGACCAAGAUGAAGUUUCA | 16 | UGAAACUUCAUCUUGGUCUCU |
| TJR102831 | 8 | UCAUCAGCUUUGCUACUGU | 17 | ACAGUAGCAAAGCUGAUGACC |
| TJR102676 | 9 | UAAAAAGGAGAAAGAAAAU | 18 | AUUUUCUUUCUCCUUUUUAUU |
| TJR102665 | 54 | AUACUAACAUGUUAUUUGA | 158 | UCAAAUAACAUGUUAGUAUUA |
| TJR102666 | 116 | CUAACAUGUUAUUUGCCUUUU | 220 | AAAAGGCAAAUAACAUGUUAGUA |
| TJR102669 | 112 | ACUUUCUUAAUAAAAAGGA | 216 | UCCUUUUUAUUAAGAAAGUAU |
| TJR102670 | 113 | UUUCUUAAUAAAAAGGAGA | 217 | UCUCCUUUUUAUUAAGAAAGU |
| TJR102671 | 114 | UUCUUAAUAAAAAGGAGAA | 218 | UUCUCCUUUUUAUUAAGAAAG |
| TJR102675 | 115 | AAUAAAAAGGAGAAAGAAA | 219 | UUUCUUUCUCCUUUUUAUUAA |
| TJR102827 | 117 | UAAUGGCAAUGUGGUCAAG | 221 | CUUGACCACAUUGCCAUUA |
| TJR102829 | 118 | UAAUGGCAAUGUGGUCAAA | 222 | UUUGACCACAUUGCCAUUA |
| TJR102836 | 121 | AGACCAAGAUGAAGUUUCC | 225 | GGAAACUUCAUCUUGGUCU |
| TJR102838 | 122 | AGACCAAGAUGAAGUUUCA | 226 | GGAAACUUCAUCUUGGUCU |
| TJR102832 | 119 | GGUCAUCAGCUUUGCUACU | 223 | AGUAGCAAAGCUGAUGACC |
| TJR102833 | 120 | UCAUCAGCUUUGCUACUGU | 224 | ACAGUAGCAAAGCUGAUGA |

**Table 1b. Unmodified sense strands and antisense strands**

| SEQ ID NO: | Unmodified SS strand (5'-3') | SEQ ID NO: | Unmodified AS strand (5'-3') |
|---|---|---|---|
| 19 | GCUAGCCAAGCAGCAAAUA | 123 | UAUUUGCUGCUUGGCUAGCUC |
| 20 | CUAGCCAAGCAGCAAAUCA | 124 | UGAUUUGCUGCUUGGCUAGCU |
| 21 | UAGCCAAGCAGCAAAUCCU | 125 | AGGAUUUGCUGCUUGGCUAGC |
| 22 | UUUGCUACUGUCACAGACU | 126 | AGUCUGUGACAGUAGCAAAGC |
| 23 | UCCUGAAACUGCAACUAGA | 127 | UCUAGUUGCAGUUUCAGGACA |
| 24 | CCUUCCUAGAGCUUAAGAU | 128 | AUCUUAAGCUCUAGGAAGGGC |
| 25 | UUAAGAUCCGAGCCAAUGA | 129 | UCAUUGGCUCGGAUCUUAAGC |
| 26 | GUACCAGCUGAAUUACUGA | 130 | UCAGUAAUUCAGCUGGUACCC |
| 27 | UGCUGCCUCUUUCCAUUCU | 131 | AGAAUGGAAAGAGGCAGCAAU |
| 28 | UCCUCAAAGCCAACAAUCA | 132 | UGAUUGUUGGCUUUGAGGAGG |
| 29 | AAUGGCAAUGUGGUCAAGA | 133 | UCUUGACCACAUUGCCAUUAU |
| 30 | GGAGUGAAGAGACCAAGAU | 134 | AUCUUGGUCUCUUCACUCCAA |
| 31 | UGAAGAGACCAAGAUGAAA | 135 | UUUCAUCUUGGUCUCUUCACU |
| 32 | GAAGAGACCAAGAUGAAGU | 136 | ACUUCAUCUUGGUCUCUUCAC |
| 33 | AAUGGGCACUUUCUUGUCU | 137 | AGACAAGAAAGUGCCCAUUUG |
| 34 | CUUUCUUGUCUGAGACUCU | 138 | AGAGUCUCAGACAAGAAAGUG |
| 35 | GAGGGAAGGCAGAGAAAAA | 139 | UUUUUCUCUGCCUUCCCUCCC |
| 36 | GGAAGGCAGAGAAAAAUUA | 140 | UAAUUUUUCUCUGCCUUCCCU |
| 37 | UCUCCCAAGAUGAGAAAGU | 141 | ACUUUCUCAUCUUGGGAGAGG |
| 38 | GGAGGAAGCAGAUAGAUGA | 142 | UCAUCUAUCUGCUUCCUCCUC |
| 39 | UAAGAAGUUCCCUGGUUUU | 143 | AAAACCAGGGAACUUCUUAGG |
| 40 | AGAAGUUCCCUGGUUUUUA | 144 | UAAAAACCAGGGAACUUCUUA |
| 41 | CCCACUGGGAGACAAGCAU | 145 | AUGCUUGUCUCCCAGUGGGUC |
| 42 | CUGGGAGACAAGCAUUUAU | 146 | AUAAAUGCUUGUCUCCCAGUG |
| 43 | GGGAGACAAGCAUUUAUAA | 147 | UUAUAAAUGCUUGUCUCCCAG |
| 44 | GGAGACAAGCAUUUAUACU | 148 | AGUAUAAAUGCUUGUCUCCCA |
| 45 | GAGACAAGCAUUUAUACUU | 149 | AAGUAUAAAUGCUUGUCUCCC |
| 46 | AGACAAGCAUUUAUACUUU | 150 | AAAGUAUAAAUGCUUGUCUCC |
| 47 | ACAAGCAUUUAUACUUUCU | 151 | AGAAAGUAUAAAUGCUUGUCU |
| 48 | CAAGCAUUUAUACUUUCUU | 152 | AAGAAAGUAUAAAUGCUUGUC |
| 49 | AAGCAUUUAUACUUUCUUU | 153 | AAAGAAAGUAUAAAUGCUUGU |
| 50 | CCACUAAUUUUUGUAUUCU | 154 | AGAAUACAAAAAUUAGUGGGC |
| 51 | CACUAAUUUUUGUAUUCUU | 155 | AAGAAUACAAAAAUUAGUGGG |
| 52 | UAAUUUUUGUAUUCUUAGU | 156 | ACUAAGAAUACAAAAAUUAGU |
| 53 | UAUACUUUCUUAAUAAAAA | 157 | UUUUUAUUAAGAAAGUAUAAG |
| 55 | ACAUGUUAUUUGCCUUUUA | 159 | UAAAAGGCAAAUAACAUGUUA |
| 56 | UUAUUUGCCUUUUGAAUUA | 160 | UAAUUCAAAAGGCAAAUAACA |
| 57 | UUUUGAAUUCUCAUUAUCU | 161 | AGAUAAUGAGAAUUCAAAAGG |
| 58 | AAUUCUCAUUAUCUUAAAA | 162 | UUUUAAGAUAAUGAGAAUUCA |
| 59 | CUCAUUAUCUUAAAAUUGU | 163 | ACAAUUUUAAGAUAAUGAGAA |
| 60 | GUGACAUGUGAUUACAUCA | 164 | UGAUGUAAUCACAUGUCACAC |
| 61 | ACAUGUGAUUACAUCAUCU | 165 | AGAUGAUGUAAUCACAUGUCA |
| 62 | AUGUGAUUACAUCAUCUUU | 166 | AAAGAUGAUGUAAUCACAUGU |
| 63 | UCAUCUUUCUGACAUCAUU | 167 | AAUGAUGUCAGAAAGAUGAUG |
| 64 | UUCUGACAUCAUUGUUAAU | 168 | AUUAACAAUGAUGUCAGAAAG |
| 65 | CAUUGUUAAUGGAAUGUGU | 169 | ACACAUUCCAUUAACAAUGAU |
| 66 | UAAUGGAAUGUGUGCUUGU | 170 | ACAAGCACACAUUCCAUUAAC |
| 67 | UUGGCCCCCAGCAAUCAGA | 171 | UCUGAUUGCUGGGGGCCAAUG |
| 68 | GCCUGCGACCCCCUUAUGU | 172 | ACAUAAGGGGGUCGCAGGCUC |
| 69 | UGCGACCCCCUUAUGUUGA | 173 | UCAACAUAAGGGGGUCGCAGG |
| 70 | UUAUGUUGCAGGCGAGACA | 174 | UGUCUCGCCUGCAACAUAAGG |
| 71 | GGGAAGGCAGAGAAAAAUU | 175 | AAUUUUUCUCUGCCUUCCCUC |
| 72 | GAAGGCAGAGAAAAAUUAA | 176 | UUAAUUUUUCUCUGCCUUCCC |
| 73 | CAAAGCUAUUUUCAUAAUA | 177 | UAUUAUGAAAAUAGCUUUGAC |
| 74 | AAAGCUAUUUUCAUAAUAA | 178 | UUAUUAUGAAAAUAGCUUUGA |
| 75 | CUAUUUUCAUAAUAAUACU | 179 | AGUAUUAUUAUGAAAAUAGCU |
| 76 | UAUUUUCAUAAUAAUACUA | 180 | UAGUAUUAUUAUGAAAAUAGC |
| 77 | AUUUUCAUAAUAAUACUAA | 181 | UUAGUAUUAUUAUGAAAAUAG |
| 78 | UUUCAUAAUAAUACUAACA | 182 | UGUUAGUAUUAUUAUGAAAAU |
| 79 | CAUAAUAAUACUAACAUGU | 183 | ACAUGUUAGUAUUAUUAUGAA |
| 80 | AAUAAUACUAACAUGUUAU | 184 | AUAACAUGUUAGUAUUAUUAU |
| 81 | UAUUUGCCUUUUGAAUUCU | 185 | AGAAUUCAAAAGGCAAAUAAC |
| 82 | UCUCAUUAUCUUAAAAUUA | 186 | UAAUUUUAAGAUAAUGAGAAU |
| 83 | CAUUAUCUUAAAAUUGUAU | 187 | AUACAAUUUUAAGAUAAUGAG |
| 84 | UUAAUGGAAUGUGUGCUUA | 188 | UAAGCACACAUUCCAUUAACA |
| 85 | UGGAGCAGGCCGGGCCAGA | 189 | UCUGGCCCGGCCUGCUCCAGG |
| 86 | GGAGCAGGCCGGGCCAGGA | 190 | UCCUGGCCCGGCCUGCUCCAG |
| 87 | GAGGAGGACCCCCACCUGU | 191 | ACAGGUGGGGGUCCUCCUCCU |
| 88 | AGGAGGACCCCCACCUGUA | 192 | UACAGGUGGGGGUCCUCCUCC |
| 89 | GGAGGACCCCCACCUGUGA | 193 | UCACAGGUGGGGGUCCUCCUC |
| 90 | GAGGACCCCCACCUGUGAA | 194 | UUCACAGGUGGGGGUCCUCCU |
| 91 | ACCUGUGAGCCUGCGACCA | 195 | UGGUCGCAGGCUCACAGGUGG |
| 92 | CCUGUGAGCCUGCGACCCA | 196 | UGGGUCGCAGGCUCACAGGUG |
| 93 | CUGUGAGCCUGCGACCCCA | 197 | UGGGGUCGCAGGCUCACAGGU |
| 94 | GCAGCCCGAGGGGUACCAA | 198 | UUGGUACCCCUCGGGCUGCAG |
| 95 | CAGCCCGAGGGGUACCAGA | 199 | UCUGGUACCCCUCGGGCUGCA |
| 96 | AGCCCGAGGGGUACCAGCU | 200 | AGCUGGUACCCCUCGGGCUGC |
| 97 | GCCCGAGGGGUACCAGCUA | 201 | UAGCUGGUACCCCUCGGGCUG |
| 98 | CCCGAGGGGUACCAGCUGA | 202 | UCAGCUGGUACCCCUCGGGCU |
| 99 | CCGAGGGGUACCAGCUGAA | 203 | UUCAGCUGGUACCCCUCGGGC |
| 100 | GGCAGUGCCCUCCCCACCU | 204 | AGGUGGGGAGGGCACUGCCCA |
| 101 | GCAGUGCCCUCCCCACCUA | 205 | UAGGUGGGGAGGGCACUGCCC |
| 102 | CAGUGCCCUCCCCACCUGA | 206 | UCAGGUGGGGAGGGCACUGCC |
| 103 | GUCAAAGCUAUUUUCAUAAUA | 207 | UAUUAUGAAAAUAGCUUUGACUU |
| 104 | GUCAAAGCUAUUUUCAUAAUA | 208 | UAUUAUGAAAAUAGCUUUGAC |
| 105 | UCAAAGCUAUUUUCAUAAUAA | 209 | UUAUUAUGAAAAUAGCUUUGACU |
| 106 | UAAUAAUACUAACAUGUUU | 210 | AAACAUGUUAGUAUUAUUAUG |
| 107 | UCAAAGCUAUUUUCAUAAA | 211 | UUUAUGAAAAUAGCUUUGACU |
| 108 | AGUCAAAGCUAUUUUCAUAAU | 212 | AUUAUGAAAAUAGCUUUGACUUU |
| 109 | CAUAAUAAUACUAACAUGUUA | 213 | UAACAUGUUAGUAUUAUUAUG |
| 110 | AGUCAAAGCUAUUUUCAUAAU | 214 | AUUAUGAAAAUAGCUUUGACU |
| 111 | GCUAUUUUCAUAAUAAUACUA | 215 | UAGUAUUAUUAUGAAAAUAGCUU |

**Table 2a. Modified sense strands and antisense strands**

| Double strand No. | SEQ ID NO: | SS strand (5'-3') | SEQ ID NO: | AS strand (5'-3') |
|---|---|---|---|---|
| TJR100 817 | 227 | UmsCmsAmAmAmGmCfUfAf UmUmUmUmCmAmUmAmA mUm | 251 | AmsUfsUmAmUmGfAmAmAmA mUmAfGmCfUmUfUmGmAmsC msUm |
| TJR101 676 | 228 | UmsCmsAmAmAmGmCfUfAf UmUmUmUmCmAmUmAmA mAm | 252 | UmsUfsUmAmUmGfAmAmAmA mUmAfGmCfUmUfUmGmAmsC msUm |
| TJR102 928 | 229 | UmsCmsAmAmAfGmCfUfAfU mUmUmUmCmAmUmAmAm Um | 253 | AmsUfsUmAmUmGfAmAmAmA mUmAmGmCfUmUfUmGmAms CmsUm |
| TJR100 532 | 230 | UmsAmsAmUmAmAmUfAfCf UmAmAmCmAmUmGmUmU mAm | 254 | UmsAfsAmCmAmUfGmUmUmA mGmUfAmUfUmAfUmUmAmsU msGm |
| TJR101 675 | 231 | UmsAmsAmUmAmAmUfAfCf UmAmAmCmAmUmGmUmU mUm | 255 | AmsAfsAmCmAmUfGmUmUmA mGmUfAmUfUmAfUmUmAmsU msGm |
| TJR100 534 | 232 | UmsUmsAmAmUmAmAfAfAf AmGmGmAmGmAmAmAmG mAm | 256 | UmsCfsUmUmUmCfUmCmCmU mUmUfUmUfAmUfUmAmAmsG msAm |
| TJR100 825 | 233 | UmsUmsAmAmUmGmGfAfAf UmGmUmGmUmGmCmUmU mAm | 257 | UmsAfsAmGmCmAfCmAmCmA mUmUfCmCfAmUfUmAmAmsC msAm |
| TJR100 543 | 234 | AmsAmsAmAmGmGmAfGfAf AmAmGmAmAmAmAmUmC mAm | 258 | UmsGfsAmUmUmUfUmCmUmU mUmCfUmCfCmUfUmUmUmsU msAm |
| TJR102 203 | 235 | AmsAmsAmAmGmGmAfGfAf AmAmGmAmAmAmAmUmsC msAm | 259 | UmsGfsAmUmUmUfUmCmUmU mUmCfUmCfCmUfUmUmUmsU msAm |
| TJR101 194 | 236 | CmsAmsUmAmAmUmAmAm UfAfCfUmAmAmCmAmUmG mUmUmAm | 260 | UmsAfsAmCmAmUfGmUmUmA mGmUfAmUfUmAfUmUmAmU mGmsAmsAm |
| TJR100 497 | 237 | UmsAmsAmUmGmGmCfAfAf UmGmUmGmGmUmCmAmA mAm | 261 | UmsUfsUmGmAmCfCmAmCmA mUmUfGmCfCmAfUmUmAmsU msGm |
| TJR100 500 | 238 | AmsGmsAmCmCmAmAfGfAf UmGmAmAmGmUmUmUmC mAm | 262 | UmsGfsAmAmAmCfUmUmCmA mUmCfUmUfGmGfUmCmUmsC msUm |
| TJR100 499 | 239 | UmsCmsAmUmCmAmGfCfUf UmUmGmCmUmAmCmUmGm Um | 263 | AmsCfsAmGmUmAfGmCmAmA mAmGfCmUfGmAfUmGmAmsC msCm |
| TJR102 204 | 240 | UmsAmsAmAmAmAmGfGfAf GmAmAmAmGmAmAmAmsA msUm | 264 | AmsUfsUmUmUmCfUmUmUmC mUmCfCmUfUmUfUmUmAmsU msUm |
| TJR102 695 | 241 | UmsAmsAmAmAmAmGfGfAf GmAmAmAmGmAmAmAmA mUm | 265 | AmsUfsUmUmUmCfUmUmUmC mUmCfCmUfUmUfUmUmAmsU msUm |
| TJR102 931 | 242 | UmsAmsAmAmAfAmGfGfAfG mAmAm AmGmAm AmAmAm Um | 266 | AmsUfsUmUfUmCfUmUmUmCf UmCf CmUfUmUfUmUf AmsUmsUm |
| TJR102 154 | 243 | UmsCmsAmAmAmGmCfUfAf UmUmUmUmCmAmUmAmA msUm-NAG0052' | 267 | AmsUfsUmAmUmGfAmAmAmA mUmAfGmCfUmUfUmGmAmsC msUm |
| TJR102 322 | 244 | UmsCmsAmAmAmGmCfUfAf UmUmUmUmCmAmUmAmA msUm-NAG0052' | 268 | AmsUfsUmAfUmGfAmAmAmAf UmAf GmCfUmUfUmGfAmsCmsUm |
| TJR102 152 | 245 | UmsAmsAmUmAmAmUfAfCf UmAmAmCmAmUmGmUmU msAm-NAG0052' | 269 | UmsAfsAmCmAmUfGmUmUmA mGmUfAmUfUmAfUmUmAmsU msGm |
| TJR102 320 | 246 | UmsAmsAmUmAmAmUfAfCf UmAmAmCmAmUmGmUmU msAm-NAG0052' | 270 | UmsAfsAmCfAmUfGmUmUmAf GmUf AmUfUmAfUmUfAmsUmsGm |
| TJR102 926 | 247 | UmsUmsAmAmUfAmAfAfAfA mGmGmAmGmAmAmAmGm Am-L96' | 271 | UmsCfsUmUfUmCfUmCmCmUf UmUf UmUfAmUfUmAfAmsGmsAm |
| TJR102 927 | 248 | AmsAmsAmAmGfGmAfGfAfA mAGmAmAmAmAmUmCm Am-L96' | 272 | UmsGfsAmUfUmUfUmCmUmUf UmCf UmCfCmUfUmUf UmsUmsAm |
| TJR102 925 | 249 | CmsAmsUmAmAfUmAfAfUfA mCmUmAmAmCmAmUmGmU mUmAm-L96' | 273 | UmsAfsAmCfAmUfGmUmUmAf GmUfAmUfUmAfUmUfAmUmG msAmsAm |
| TJR102 924 | 250 | UmsAmsAmAmAfAmGfGfAfG mAmAmAmGmAmAmAmAm Um-L96’ | 274 | AmsUfsUmUmUmCfUmUmUmC mUmCmCmUfUmUfUmUmAms UmsUm |

**Table 2b. Modified sense strands and antisense strands**

| Double strand No. | SEQ ID NO: | SS strand (5'-3') | SEQ ID NO: | AS strand (5'-3') |
|---|---|---|---|---|
| TJR1004 94 | 275 | GmsCmsUmAmGmCmCfAf AfGmCmAmGmCmAmAm AmUmAm | 384 | UmsAfsUmUmUmGfCmUmGm CmUmUfGmGfCmUfAmGmCm sUmsCm |
| TJR1004 95 | 276 | CmsUmsAmGmCmCmAfAf GfCmAmGmCmAmAmAm UmCmAm | 385 | UmsGfsAmUmUmUfGmCmUm GmCmUfUmGfGmCfUmAmGm sCmsUm |
| TJR1004 96 | 277 | UmsAmsGmCmCmAmAfGf CfAmGmCmAmAmAmUmC mCmUm | 386 | AmsGfsGmAmUmUfUmGmCm UmGmCfUmUfGmGfCmUmAm sGmsCm |
| TJR1005 03 | 278 | UmsUmsUmGmCmUmAfCf UfGmUmCmAmCmAmGm AmCmUm | 387 | AmsGfsUmCmUmGfUmGmAm CmAmGfUmAfGmCfAmAmAm sGmsCm |
| TJR1005 04 | 279 | UmsCmsCmUmGmAmAfAf CfUmGmCmAmAmCmUmA mGmAm | 388 | UmsCfsUmAmGmUfUmGmCm AmGmUfUmUfCmAfGmGmA msCmsAm |
| TJR1005 09 | 280 | CmsCmsUmUmCmCmUfAf GfAmGmCmUmUmAmAm GmAmUm | 389 | AmsUfsCmUmUmAfAmGmCm UmCmUfAmGfGmAfAmGmG msGmsCm |
| TJR1005 01 | 281 | UmsUmsAmAmGmAmUfCf CfGmAmGmCmCmAmAmU mGmAm | 390 | UmsCfsAmUmUmGfGmCmUm CmGmGfAmUfCmUfUmAmAm sGmsCm |
| TJR1005 02 | 282 | GmsUmsAmCmCmAmGfCf UfGmAmAmUmUmAmCm UmGmAm | 391 | UmsCfsAmGmUmAfAmUmUm CmAmGfCmUfGmGfUmAmCm sCmsCm |
| TJR1005 05 | 283 | UmsGmsCmUmGmCmCfUf CfUmUmUmCmCmAmUmU mCmUm | 392 | AmsGfsAmAmUmGfGmAmAm AmGmAfGmGfCmAfGmCmAm sAmsUm |
| TJR1005 10 | 284 | UmsCmsCmUmCmAmAfAf GfCmCmAmAmCmAmAmU mCmAm | 393 | UmsGfsAmUmUmGfUmUmGm GmCmUfUmUfGmAfGmGmA msGmsGm |
| TJR1004 98 | 285 | AmsAmsUmGmGmCmAfAf UfGmUmGmGmUmCmAm AmGmAm | 394 | UmsCfsUmUmGmAfCmCmAm CmAmUfUmGfCmCfAmUmUm sAmsUm |
| TJR1005 11 | 286 | GmsGmsAmGmUmGmAfAf GfAmGmAmCmCmAmAm GmAmUm | 395 | AmsUfsCmUmUmGfGmUmCm UmCmUfUmCfAmCfUmCmCm sAmsAm |
| TJR1005 06 | 287 | UmsGmsAmAmGmAmGfAf CfCmAmAmGmAmUmGm AmAmAm | 396 | UmsUfsUmCmAmUfCmUmUm GmGmUfCmUfCmUfUmCmAm sCmsUm |
| TJR1005 12 | 288 | GmsAmsAmGmAmGmAfCf CfAmAmGmAmUmGmAm AmGmUm | 397 | AmsCfsUmUmCmAfUmCmUm UmGmGfUmCfUmCfUmUmCm sAmsCm |
| TJR1005 07 | 289 | AmsAmsUmGmGmGmCfAf CfUmUmUmCmUmUmGm UmCmUm | 398 | AmsGfsAmCmAmAfGmAmAm AmGmUfGmCfCmCfAmUmUm sUmsGm |
| TJR1005 08 | 290 | CmsUmsUmUmCmUmUfGf UfCmUmGmAmGmAmCm UmCmUm | 399 | AmsGfsAmGmUmCfUmCmAm GmAmCfAmAfGmAfAmAmG msUmsGm |
| TJR1005 35 | 291 | GmsAmsGmGmGmAmAfGf GfCmAmGmAmGmAmAm AmAmAm | 400 | UmsUfsUmUmUmCfUmCmUm GmCmCfUmUfCmCfCmUmCm sCmsCm |
| TJR1005 36 | 292 | GmsGmsAmAmGmGmCfAf GfAmGmAmAmAmAmAm UmUmAm | 401 | UmsAfsAmUmUmUfUmUmCm UmCmUfGmCfCmUfUmCmCm sCmsUm |
| TJR1005 37 | 293 | UmsCmsUmCmCmCmAfAf GfAmUmGmAmGmAmAm AmGmUm | 402 | AmsCfsUmUmUmCfUmCmAm UmCmUfUmGfGmGfAmGmA msGmsGm |
| TJR1005 18 | 294 | GmsGmsAmGmGmAmAfGf CfAmGmAmUmAmGmAm UmGmAm | 403 | UmsCfsAmUmCmUfAmUmCm UmGmCfUmUfCmCfUmCmCm sUmsCm |
| TJR1005 19 | 295 | UmsAmsAmGmAmAmGfUf UfCmCmCmUmGmGmUmU mUmUm | 404 | AmsAfsAmAmCmCfAmGmGm GmAmAfCmUfUmCfUmUmAm sGmsGm |
| TJR1005 38 | 296 | AmsGmsAmAmGmUmUfCf CfCmUmGmGmUmUmUm UmUmAm | 405 | UmsAfsAmAmAmAfCmCmAm GmGmGfAmAfCmUfUmCmUm sUmsAm |
| TJR1005 30 | 297 | CmsCmsCmAmCmUmGfGf GfAmGmAmCmAmAmGm CmAmUm | 406 | AmsUfsGmCmUmUfGmUmCm UmCmCfCmAfGmUfGmGmGm sUmsCm |
| TJR1005 20 | 298 | CmsUmsGmGmGmAmGfAf CfAmAmGmCmAmUmUm UmAmUm | 407 | AmsUfsAmAmAmUfGmCmUm UmGmUfCmUfCmCfCmAmGm sUmsGm |
| TJR1005 39 | 299 | GmsGmsGmAmGmAmCfAf AfGmCmAmUmUmUmAm UmAmAm | 408 | UmsUfsAmUmAmAfAmUmGm CmUmUfGmUfCmUfCmCmCm sAmsGm |
| TJR1005 40 | 300 | GmsGmsAmGmAmCmAfAf GfCmAmUmUmUmAmUm AmCmUm | 409 | AmsGfsUmAmUmAfAmAmUm GmCmUfUmGfUmCfUmCmCm sCmsAm |
| TJR1005 21 | 301 | GmsAmsGmAmCmAmAfGf CfAmUmUmUmAmUmAm CmUmUm | 410 | AmsAfsGmUmAmUfAmAmAm UmGmCfUmUfGmUfCmUmCm sCmsCm |
| TJR1005 16 | 302 | AmsGmsAmCmAmAmGfCf AfUmUmUmAmUmAmCm UmUmUm | 411 | AmsAfsAmGmUmAfUmAmAm AmUmGfCmUfUmGfUmCmUm sCmsCm |
| TJR1005 17 | 303 | AmsCmsAmAmGmCmAfUf UfUmAmUmAmCmUmUm UmCmUm | 412 | AmsGfsAmAmAmGfUmAmUm AmAmAfUmGfCmUfUmGmU msCmsUm |
| TJR1005 13 | 304 | CmsAmsAmGmCmAmUfUf UfAmUmAmCmUmUmUm CmUmUm | 413 | AmsAfsGmAmAmAfGmUmAm UmAmAfAmUfGmCfUmUmG msUmsCm |
| TJR1005 41 | 305 | AmsAmsGmCmAmUmUfUf AfUmAmCmUmUmUmCm UmUmUm | 414 | AmsAfsAmGmAmAfAmGmUm AmUmAfAmAfUmGfCmUmU msGmsUm |
| TJR1005 22 | 306 | CmsCmsAmCmUmAmAfUf UfUmUmUmGmUmAmUm UmCmUm | 415 | AmsGfsAmAmUmAfCmAmAm AmAmAfUmUfAmGfUmGmG msGmsCm |
| TJR1005 31 | 307 | CmsAmsCmUmAmAmUfUf UfUmUmGmUmAmUmUm CmUmUm | 416 | AmsAfsGmAmAmUfAmCmAm AmAmAfAmUfUmAfGmUmG msGmsGm |
| TJR1005 23 | 308 | UmsAmsAmUmUmUmUfUf GfUmAmUmUmCmUmUm AmGmUm | 417 | AmsCfsUmAmAmGfAmAmUm AmCmAfAmAfAmAfUmUmA msGmsUm |
| TJR1005 42 | 309 | UmsAmsUmAmCmUmUfUf CfUmUmAmAmUmAmAm AmAmAm | 418 | UmsUfsUmUmUmAfUmUmAm AmGmAfAmAfGmUfAmUmA msAmsGm |
| TJR1005 33 | 310 | AmsUmsAmCmUmAmAfCf AfUmGmUmUmAmUmUm UmGmAm | 419 | UmsCfsAmAmAmUfAmAmCm AmUmGfUmUfAmGfUmAmU msUmsAm |
| TJR1005 24 | 311 | AmsCmsAmUmGmUmUfAf UfUmUmGmCmCmUmUm UmUmAm | 420 | UmsAfsAmAmAmGfGmCmAm AmAmUfAmAfCmAfUmGmU msUmsAm |
| TJR1005 44 | 312 | UmsUmsAmUmUmUmGfCf CfUmUmUmUmGmAmAm UmUmAm | 421 | UmsAfsAmUmUmCfAmAmAm AmGmGfCmAfAmAfUmAmA msCmsAm |
| TJR1005 25 | 313 | UmsUmsUmUmGmAmAfUf UfCmUmCmAmUmUmAm UmCmUm | 422 | AmsGfsAmUmAmAfUmGmAm GmAmAfUmUfCmAfAmAmA msGmsGm |
| TJR1005 45 | 314 | AmsAmsUmUmCmUmCfAf UfUmAmUmCmUmUmAm AmAmAm | 423 | UmsUfsUmUmAmAfGmAmUm AmAmUfGmAfGmAfAmUmU msCmsAm |
| TJR1005 46 | 315 | CmsUmsCmAmUmUmAfUf CfUmUmAmAmAmAmUm UmGmUm | 424 | AmsCfsAmAmUmUfUmUmAm AmGmAfUmAfAmUfGmAmG msAmsAm |
| TJR1005 47 | 316 | GmsUmsGmAmCmAmUfGf UfGmAmUmUmAmCmAm UmCmAm | 425 | UmsGfsAmUmGmUfAmAmUm CmAmCfAmUfGmUfCmAmCm sAmsCm |
| TJR1005 14 | 317 | AmsCmsAmUmGmUmGfAf UfUmAmCmAmUmCmAm UmCmUm | 426 | AmsGfsAmUmGmAfUmGmUm AmAmUfCmAfCmAfUmGmUm sCmsAm |
| TJR1005 26 | 318 | AmsUmsGmUmGmAmUfUf AfCmAmUmCmAmUmCmU mUmUm | 427 | AmsAfsAmGmAmUfGmAmUm GmUmAfAmUfCmAfCmAmUm sGmsUm |
| TJR1005 15 | 319 | UmsCmsAmUmCmUmUfUf CfUmGmAmCmAmUmCmA mUmUm | 428 | AmsAfsUmGmAmUfGmUmCm AmGmAfAmAfGmAfUmGmA msUmsGm |
| TJR1005 27 | 320 | UmsUmsCmUmGmAmCfAf UfCmAmUmUmGmUmUm AmAmUm | 429 | AmsUfsUmAmAmCfAmAmUm GmAmUfGmUfCmAfGmAmA msAmsGm |
| TJR1005 28 | 321 | CmsAmsUmUmGmUmUfAf AfUmGmGmAmAmUmGm UmGmUm | 430 | AmsCfsAmCmAmUfUmCmCm AmUmUfAmAfCmAfAmUmG msAmsUm |
| TJR1005 29 | 322 | UmsAmsAmUmGmGmAfAf UfGmUmGmUmGmCmUm UmGmUm | 431 | AmsCfsAmAmGmCfAmCmAm CmAmUfUmCfCmAfUmUmAm sAmsCm |
| TJR1008 12 | 323 | UmsUmsGmGmCmCmCfCf CfAmGmCmAmAmUmCmA mGmAm | 432 | UmsCfsUmGmAmUfUmGmCm UmGmGfGmGfGmCfCmAmAm sUmsGm |
| TJR1008 13 | 324 | GmsCmsCmUmGmCmGfAf CfCmCmCmCmUmUmAmU mGmUm | 433 | AmsCfsAmUmAmAfGmGmGm GmGmUfCmGfCmAfGmGmCm sUmsCm |
| TJR1008 14 | 325 | UmsGmsCmGmAmCmCfCf CfCmUmUmAmUmGmUm UmGmAm | 434 | UmsCfsAmAmCmAfUmAmAm GmGmGfGmGfUmCfGmCmAm sGmsGm |
| TJR1008 15 | 326 | UmsUmsAmUmGmUmUfGf CfAmGmGmCmGmAmGm AmCmAm | 435 | UmsGfsUmCmUmCfGmCmCm UmGmCfAmAfCmAfUmAmAm sGmsGm |
| TJR1007 29 | 327 | GmsGmsGmAmAmGmGfCf AfGmAmGmAmAmAmAm AmUmUm | 436 | AmsAfsUmUmUmUfUmCmUm CmUmGfCmCfUmUfCmCmCm sUmsCm |
| TJR1008 16 | 328 | GmsAmsAmGmGmCmAfGf AfGmAmAmAmAmAmUm UmAmAm | 437 | UmsUfsAmAmUmUfUmUmUm CmUmCfUmGfCmCfUmUmCm sCmsCm |
| TJR1008 18 | 329 | CmsAmsAmAmGmCmUfAf UfUmUmUmCmAmUmAm AmUmAm | 438 | UmsAfsUmUmAmUfGmAmAm AmAmUfAmGfCmUfUmUmG msAmsCm |
| TJR1008 19 | 330 | AmsAmsAmGmCmUmAfUf UfUmUmCmAmUmAmAm UmAmAm | 439 | UmsUfsAmUmUmAfUmGmAm AmAmAfUmAfGmCfUmUmU msGmsAm |
| TJR1008 20 | 331 | CmsUmsAmUmUmUmUfCf AfUmAmAmUmAmAmUm AmCmUm | 440 | AmsGfsUmAmUmUfAmUmUm AmUmGfAmAfAmAfUmAmG msCmsUm |
| TJR1008 21 | 332 | UmsAmsUmUmUmUmCfAf UfAmAmUmAmAmUmAm CmUmAm | 441 | UmsAfsGmUmAmUfUmAmUm UmAmUfGmAfAmAfAmUmA msGmsCm |
| TJR1007 39 | 333 | AmsUmsUmUmUmCmAfUf AfAmUmAmAmUmAmCm UmAmAm | 442 | UmsUfsAmGmUmAfUmUmAm UmUmAfUmGfAmAfAmAmU msAmsGm |
| TJR1007 40 | 334 | UmsUmsUmCmAmUmAfAf UfAmAmUmAmCmUmAm AmCmAm | 443 | UmsGfsUmUmAmGfUmAmUm UmAmUfUmAfUmGfAmAmA msAmsUm |
| TJR1007 41 | 335 | CmsAmsUmAmAmUmAfAf UfAmCmUmAmAmCmAm UmGmUm | 444 | AmsCfsAmUmGmUfUmAmGm UmAmUfUmAfUmUfAmUmG msAmsAm |
| TJR1007 42 | 336 | AmsAmsUmAmAmUmAfCf UfAmAmCmAmUmGmUm UmAmUm | 445 | AmsUfsAmAmCmAfUmGmUm UmAmGfUmAfUmUfAmUmU msAmsUm |
| TJR1008 22 | 337 | UmsAmsUmUmUmGmCfCf UfUmUmUmGmAmAmUm UmCmUm | 446 | AmsGfsAmAmUmUfCmAmAm AmAmGfGmCfAmAfAmUmA msAmsCm |
| TJR1008 23 | 338 | UmsCmsUmCmAmUmUfAf UfCmUmUmAmAmAmAm UmUmAm | 447 | UmsAfsAmUmUmUfUmAmAm GmAmUfAmAfUmGfAmGmA msAmsUm |
| TJR1008 24 | 339 | CmsAmsUmUmAmUmCfUf UfAmAmAmAmUmUmGm UmAmUm | 448 | AmsUfsAmCmAmAfUmUmUm UmAmAfGmAfUmAfAmUmG msAmsGm |
| TJR1008 30 | 340 | UmsGmsGmAmGmCmAfGf GfCmCmGmGmGmCmCmA mGmAm | 449 | UmsCfsUmGmGmCfCmCmGm GmCmCfUmGfCmUfCmCmAm sGmsGm |
| TJR1008 31 | 341 | GmsGmsAmGmCmAmGfGf CfCmGmGmGmCmCmAmG mGmAm | 450 | UmsCfsCmUmGmGfCmCmCm GmGmCfCmUfGmCfUmCmCm sAmsGm |
| TJR1008 32 | 342 | GmsAmsGmGmAmGmGfAf CfCmCmCmCmAmCmCmU mGmUm | 451 | AmsCfsAmGmGmUfGmGmGm GmGmUfCmCfUmCfCmUmCm sCmsUm |
| TJR1008 33 | 343 | AmsGmsGmAmGmGmAfCf CfCmCmCmAmCmCmUmG mUmAm | 452 | UmsAfsCmAmGmGfUmGmGm GmGmGfUmCfCmUfCmCmUm sCmsCm |
| TJR1008 34 | 344 | GmsGmsAmGmGmAmCfCf CfCmCmAmCmCmUmGmU mGmAm | 453 | UmsCfsAmCmAmGfGmUmGm GmGmGfGmUfCmCfUmCmCm sUmsCm |
| TJR1008 35 | 345 | GmsAmsGmGmAmCmCfCf CfCmAmCmCmUmGmUmG mAmAm | 454 | UmsUfsCmAmCmAfGmGmUm GmGmGfGmGfUmCfCmUmCm sCmsUm |
| TJR1008 36 | 346 | AmsCmsCmUmGmUmGfAf GfCmCmUmGmCmGmAmC mCmAm | 455 | UmsGfsGmUmCmGfCmAmGm GmCmUfCmAfCmAfGmGmUm sGmsGm |
| TJR1008 37 | 347 | CmsCmsUmGmUmGmAfGf CfCmUmGmCmGmAmCmC mCmAm | 456 | UmsGfsGmGmUmCfGmCmAm GmGmCfUmCfAmCfAmGmGm sUmsGm |
| TJR1008 38 | 348 | CmsUmsGmUmGmAmGfCf CfUmGmCmGmAmCmCmC mCmAm | 457 | UmsGfsGmGmGmUfCmGmCm AmGmGfCmUfCmAfCmAmGm sGmsUm |
| TJR1008 39 | 349 | GmsCmsAmGmCmCmCfGf AfGmGmGmGmUmAmCm CmAmAm | 458 | UmsUfsGmGmUmAfCmCmCm CmUmCfGmGfGmCfUmGmCm sAmsGm |
| TJR1008 40 | 350 | CmsAmsGmCmCmCmGfAf GfGmGmGmUmAmCmCm AmGmAm | 459 | UmsCfsUmGmGmUfAmCmCm CmCmUfCmGfGmGfCmUmGm sCmsAm |
| TJR1008 41 | 351 | AmsGmsCmCmCmGmAfGf GfGmGmUmAmCmCmAm GmCmUm | 460 | AmsGfsCmUmGmGfUmAmCm CmCmCfUmCfGmGfGmCmUm sGmsCm |
| TJR1008 42 | 352 | GmsCmsCmCmGmAmGfGf GfGmUmAmCmCmAmGmC mUmAm | 461 | UmsAfsGmCmUmGfGmUmAm CmCmCfCmUfCmGfGmGmCm sUmsGm |
| TJR1008 43 | 353 | CmsCmsCmGmAmGmGfGf GfUmAmCmCmAmGmCmU mGmAm | 462 | UmsCfsAmGmCmUfGmGmUm AmCmCfCmCfUmCfGmGmGm sCmsUm |
| TJR1008 44 | 354 | CmsCmsGmAmGmGmGfGf UfAmCmCmAmGmCmUmG mAmAm | 463 | UmsUfsCmAmGmCfUmGmGm UmAmCfCmCfCmUfCmGmGm sGmsCm |
| TJR1008 45 | 355 | GmsGmsCmAmGmUmGfCf CfCmUmCmCmCmCmAmC mCmUm | 464 | AmsGfsGmUmGmGfGmGmAm GmGmGfCmAfCmUfGmCmCm sCmsAm |
| TJR1008 46 | 356 | GmsCmsAmGmUmGmCfCf CfUmCmCmCmCmAmCmC mUmAm | 465 | UmsAfsGmGmUmGfGmGmGm AmGmGfGmCfAmCfUmGmCm sCmsCm |
| TJR1008 47 | 357 | CmsAmsGmUmGmCmCfCf UfCmCmCmCmAmCmCmU mGmAm | 466 | UmsCfsAmGmGmUfGmGmGm GmAmGfGmGfCmAfCmUmGm sCmsCm |
| TJR10119 7 | 358 | GmsUmsCmAmAmAmGmC mUfAfUfUmUmUmCmAmU mAmAmUmAm | 467 | UmsAfsUmUmAmUfGmAmAm AmAmUfAmGfCmUfUmUmG mAmCmsUmsUm |
| TJR1016 79 | 359 | GmsUmsCmAmAmAmGmC mUfAfUfUmUmUmCmAmU mAmAmUmAm | 468 | UmsAfsUmUmAmUfGmAmAm AmAmUfAmGfCmUfUmUmG msAmsCm |
| TJR10119 9 | 360 | UmsCmsAmAmAmGmCmU mAfUfUfUmUmCmAmUmA mAmUmAmAm | 469 | UmsUfsAmUmUmAfUmGmAm AmAmAfUmAfGmCfUmUmU mGmAmsCmsUm |
| TJR1016 77 | 361 | CmsAmsUmAmAmUmAmA mUfAfCfUmAmAmCmAmU mGmUmUmAm | 470 | UmsAfsAmCmAmUfGmUmUm AmGmUfAmUfUmAfUmUmA msUmsGm |
| TJR1016 78 | 362 | AmsGmsUmCmAmAmAmG mCfUfAfUmUmUmUmCmA mUmAmAmUm | 471 | AmsUfsUmAmUmGfAmAmAm AmUmAfGmCfUmUfUmGmA msCmsUm |
| TJR10119 8 | 363 | AmsGmsUmCmAmAmAmG mCfUfAfUmUmUmUmCmA mUmAmAmUm | 472 | AmsUfsUmAmUmGfAmAmAm AmUmAfGmCfUmUfUmGmA mCmUmsUmsUm |
| TJR1012 00 | 364 | GmsCmsUmAmUmUmUmU mCfAfUfAmAmUmAmAmU mAmCmUmAm | 473 | UmsAfsGmUmAmUfUmAmUm UmAmUfGmAfAmAfAmUmA mGmCmsUmsUm |
| TJR1026 90 | 365 | AmsCmsUmUmUmCmUfUf AfAmUmAmAmAmAmAm GmGmAm | 474 | UmsCfsCmUmUmUfUmUmAm UmUmAfAmGfAmAfAmGmU msAmsUm |
| TJR1026 91 | 366 | UmsUmsUmCmUmUmAfAf UfAmAmAmAmAmGmGm AmGmAm | 475 | UmsCfsUmCmCmUfUmUmUm UmAmUfUmAfAmGfAmAmA msGmsUm |
| TJR1026 92 | 367 | UmsUmsCmUmUmAmAfUf AfAmAmAmAmGmGmAm GmAmAm | 476 | UmsUfsCmUmCmCfUmUmUm UmUmAfUmUfAmAfGmAmA msAmsGm |
| TJR1026 94 | 368 | AmsAmsUmAmAmAmAfAf GfGmAmGmAmAmAmGm AmAmAm | 477 | UmsUfsUmCmUmUfUmCmUm CmCmUfUmUfUmUfAmUmUm sAmsAm |
| TJR1029 29 | 369 | CmsUmsAmUmUfUmUfCfA fUmAmAm UmAmAm UmAmCm Um | 478 | AmsGfsUmAmUmUfAmUmUm AmUmGmAmAfAmAfUmAmG msCmsUm |
| TJR1029 30 | 370 | GmsUmsCmCmAfCmAfAfA fGmUmCmAmAmAmGmC mUm AmUmUm | 479 | AmsAfsUmAmGmCfUmUmUm GmAmCmUmUfUmGfUmGmG mAmCmsAmsCm |
| TJR1029 32 | 371 | GmsAmsGmAmAmAmGfA mAfAfAfUmCmAmAm CmAmAm AmUmUm | 480 | AmsAfsUmUfUmGfUmUmGm AfUmUf UmUfCm UfUmUf CmUmCms CmsUm |
| TJR1029 33 | 372 | AmsAmsUmAmAfAmAfAf GfGmAmGmAmAmAmGm AmAm Am | 481 | UmsUfsUmCfUmUfUmCmUmC fCmUfUmUfUmUfAmUfUmsA msAm |
| TJR1028 43 | 373 | UmsAmsAmUmGmGmCfAf AfUmGmUmGmGmUmCm AmAmGm | 482 | CmsUfsUmGmAmCfCmAmCm AmUmUfGmCfCmAfUmsUmsA m |
| TJR1028 44 | 374 | UmsAmsAmUmGmGmCmA mAfUfGfUmGmGmUmCmA mAmGmAmUm | 483 | AmsUfsCmUmUmGfAmCmCm AmCmAfUmUfGmCfCmAmUm UmAmsUmsGm |
| TJR1028 45 | 375 | UmsAmsAmUmGmGmCfAf AfUmGmUmGmGmUmCm AmAmAm | 484 | UmsUfsUmGmAmCfCmAmCm AmUmUfGmCfCmAfUmsUmsA m |
| TJR1028 46 | 376 | CmsAmsUmAmAmUmGmG mCfAfAfUmGmUmGmGmU mCmAmAmAm | 485 | UmsUfsUmGmAmCfCmAmCm AmUmUfGmCfCmAfUmUmAm UmGmsAmsUm |
| TJR1028 47 | 377 | GmsGmsUmCmAmUmCfAf GfCmUmUmUmGmCmUm AmCmUm | 486 | AmsGfsUmAmGmCfAmAmAm GmCmUfGmAfUmGfAmsCmsC m |
| TJR1028 48 | 378 | UmsCmsAmUmCmAmGfCf UfUmUmGmCmUmAmCm UmGmUm | 487 | AmsCfsAmGmUmAfGmCmAm AmAmGfCmUfGmAfUmsGms Am |
| TJR1028 49 | 379 | GmsGmsUmCmAmUmCmA mGfCfUfUmUmGmCmUmA mCmUmGmUm | 488 | AmsCfsAmGmUmAfGmCmAm AmAmGfCmUfGmAfUmGmA mCmCmsUmsCm |
| TJR1028 50 | 380 | AmsGmsAmCmCmAmAfGf AfUmGmAmAmGmUmUm UmCmCm | 489 | GmsGfsAmAmAmCfUmUmCm AmUmCfUmUfGmGfUmsCmsU m |
| TJR1028 51 | 381 | AmsGmsAmGmAmCmCmA mAfGfAfUmGmAmAmGm UmUmUmCmUm | 490 | AmsGfsAmAmAmCfUmUmCm AmUmCfUmUfGmGfUmCmUm CmUmsUmsCm |
| TJR1028 52 | 382 | AmsGmsAmCmCmAmAfGf AfUmGmAmAmGmUmUm UmCmAm | 491 | UmsGfsAmAmAmCfUmUmCm AmUmCfUmUfGmGfUmsCmsU m |
| TJR1028 53 | 383 | AmsGmsAmGmAmCmCmA mAfGfAfUmGmAmAmGm UmUmUmCmAm | 492 | UmsGfsAmAmAmCfUmUmCm AmUmCfUmUfGmGfUmCmUm CmUmsUmsCm |

In above Table 2, the direction from the 5' end to the 3' end is directed by a sequence from the left to the right; the lowercase letter m indicates that a nucleoside adjacent to the left of the letter m is a 2'-methoxy-modified nucleoside; the lowercase letter f indicates that a nucleoside adjacent to the left of the letter f is a 2'-fluoro-modified nucleoside; the lowercase letter s indicates that linkage between two nucleosides flanking the letter s or between the nucleoside linked thereto and the delivery group NAG0052' or L96' is linkage via a phosphorothioate diester group; unless otherwise specified, two adjacent nucleosides are linked via a phosphodiester group; unless otherwise specified, the 3' position of the first nucleotide at the 3' end of each strand is hydroxy; the 5' position of the first nucleotide at the 5' end of each strand is hydroxy.

The structures of the 2'-methoxy-modified nucleoside, the 2'-fluoro-modified nucleoside, the phosphorothioate diester group, the phosphodiester group, NAG0052', and L96' are shown in the table below. When the siRNAs or siRNA conjugates of the present disclosure are present in the form of a salt, e.g., in the form of a sodium salt, the structures in the form of a salt corresponding to the structures in Table 3 below are also within the claimed scope of the present disclosure.

**Table 3**

| Structure | Name |
|---|---|
| 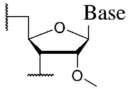 | 2'-Methoxy-modified nucleoside |
| 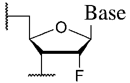 | 2'-Fluoro-modified nucleoside |
| 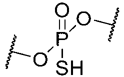 | Phosphorothioate diester group |
| 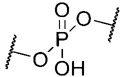 | Phosphodiester group |
| 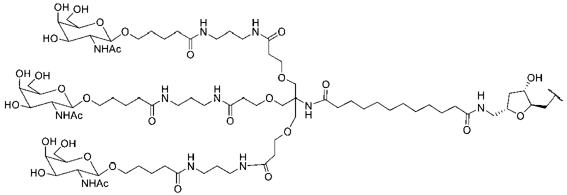 | NAG0052' |
| 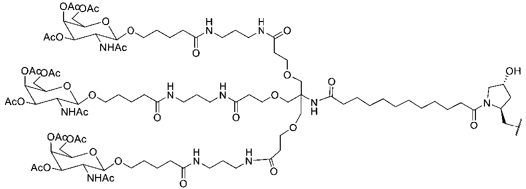 | L96' |

In those structures, Base represents a base.

### Example 2. Synthesis of siRNAs and siRNA Conjugates

The siRNA synthesis followed the conventional phosphoramidite solid-phase synthesis method. The synthesis process is briefly described below: Nucleoside phosphoramidite monomers were linked one by one according to the synthesis program on a Dr. Oligo48 synthesizer (Biolytic), starting at a universal CPG support. The nucleoside monomer starting materials 2'-F RNA, 2'-O-methyl RNA, and other nucleoside phosphoramidite monomers were purchased from Shanghai Hongene Biotech Corporationor Suzhou GenePharma Co., Ltd.. 5-Ethylthio-1H-tetrazole (ETT) was used as an activator (a 0.6 M solution in acetonitrile), a 0.22 M solution of PADS in acetonitrile and collidine (1:1 by volume) (

Suzhou Kroma BioScience, Inc.) as a sulfurizing agent, and an iodopyridine/water solution (Suzhou Kroma BioScience, Inc. as an oxidant.

After completion of solid-phase synthesis, oligoribonucleotides were cleaved from the solid support and soaked in a solution of 28% ammonia water and ethanol (3:1) at 50 °C for 16 h. The mixture was centrifuged, and the supernatant was transferred to another centrifuge tube. After the supernatant was concentrated to dryness by evaporation, the residue was purified by C18 reversed-phase chromatography using 0.1 M TEAA and acetonitrile as the mobile phase, and DMTr was removed using a 3% trifluoroacetic acid solution. The target oligonucleotides were collected, then lyophilized, identified as the target products by LC-MS, and quantified by UV (260 nm).

The resulting single-stranded oligonucleotides were complementarily paired in an equimolar ratio and annealed. The final double-stranded siRNA was dissolved in 1× PBS, and the solution was adjusted to the concentration required for the experiment and set aside for later use.

An siRNA conjugate containing NAG0052' was prepared according to the same method as described in the example of WO2023138663A1. An siRNA conjugate containing L96' was prepared according to the same method as described in the example of WO2014025805A1.

### Example 3. psiCHECK Validation of On-Target Levels

The siRNAs in Table 2 were subjected to an *in vitro* molecular-level simulation of on-target activity screening in HEK293A cells using 9 concentration gradients.

The psi-CHECK plasmids used in this example were purchased from Sangon Biotech (Shanghai) Co., Ltd. Corresponding on-target sequences of the siRNAs were constructed from the INHBE gene and inserted into the psiCHECK-2 plasmids, thereby giving the GSCM on-target plasmids. The plasmids contained the renilla luciferase gene and the firefly luciferase gene. The plasmids were dual reporter gene systems. The target sequence of the siRNA was inserted into the 3' UTR region of the renilla luciferase gene. The activity of the siRNAs for the target sequence was reflected by measuring the renilla luciferase expression after calibration with firefly luciferase. The measurement used Dual-Luciferase Reporter Assay System (Promega, E2940).

The HEK293A cells were cultured at 37 °C with 5% CO₂ in a DMEM high glucose culture medium containing 10% fetal bovine serum. 24 h prior to transfection, the HEK293A cells were seeded into a 96-well plate at a density of 8 × 10³ cells per well, with each well containing 100 µL of the culture medium.

The cells were co-transfected with the siRNAs and the corresponding plasmids using Lipofectamine2000 (ThermoFisher, 11668019) according to the instructions. 0.2 µL of Lipofectamine2000 was used for each well. The amount of plasmid for transfection was 20 ng per well. For the on-target sequence plasmids, a total of 9 concentration points were set up for the siRNAs, with the final concentration of the highest concentration point being 20 nM. A 4-fold serial dilution was performed, resulting in 20 nM, 5 nM, 1.25 nM, 0.3125 nM, 0.0781 nM, 0.0195 nM, 0.0049 nM, 0.0012 nM, and 0.0003 nM. 24 h after transfection, the on-target levels were determined using Dual-Luciferase Reporter Assay System (Promega, E2940).

The experimental materials and instruments used in this example are detailed in Table 4 and Table 5. The dilution protocol used in this example is shown in Table 6. The experimental results are shown in Table 7.

**Table 4. Experimental consumables and reagents for psi-CHECK**

| **Name** | **Company** | **Cat. No./model** |
|---|---|---|
| HEK293A cells | Nanjing Cobioer Biosciences Co., Ltd. | ATCC-Cobioer/CBP60202 |
| Dual-Glo^{®} Luciferase Assay System | Promega | E2940 |
| Lipofectamine^{®} 2000 | Invitrogen | 11668-019 |

**Table 5. Experimental instruments for psi-CHECK**

| **Experimental instrument** | | |
|---|---|---|
| **Name** | **Company** | **Cat. No./model** |
| Nanodrop | Thermo | Nanodrop One |
| Microplate reader | PerkinElmer | EnVision2105 |
| Graphing software | / | Graph Prism 5 |

**Table 6. Multi-concentration dilution protocol for samples**

| Final concentration of siRNA (nM) | Addition of water and siRNA |
|---|---|
| 20.0000 | 4 µL siRNA + 36 µL H2O |
| 5.0000 | 10 µL siRNA + 30 µL H2O |
| 1.2500 | 10 µL siRNA + 30 µL H2O |
| 0.3125 | 10 µL siRNA + 30 µL H2O |
| 0.0781 | 10 µL siRNA + 30 µL H2O |
| 0.0195 | 10 µL siRNA + 30 µL H2O |
| 0.0049 | 10 µL siRNA + 30 µL H2O |
| 0.0012 | 10 µL siRNA + 30 µL H2O |
| 0.0003 | 10 µL siRNA + 30 µL H2O |

**Table 7. psi-CHECK on-target activity screening results**

| Double strand code | GSCM IC₅₀ (nM) | Double strand code | GSCM IC₅₀ (nM) | Double strand code | GSCM IC₅₀ (nM) |
|---|---|---|---|---|---|
| TJR100545 | 0.0018 | TJR100539 | 0.0148 | TJR100494 | 0.0776 |
| TJR100544 | 0.0025 | TJR100524 | 0.0151 | TJR100523 | 0.0933 |
| TJR100526 | 0.0039 | TJR100497 | 0.0166 | TJR100505 | 0.1063 |
| TJR100515 | 0.0042 | TJR100540 | 0.0195 | TJR100495 | 0.1249 |
| TJR100527 | 0.0064 | TJR100506 | 0.0201 | TJR100537 | 0.1259 |
| TJR100525 | 0.0078 | TJR100517 | 0.0251 | TJR100510 | 0.1371 |
| TJR100514 | 0.0082 | TJR100500 | 0.0278 | TJR100511 | 0.1371 |
| TJR100529 | 0.0083 | TJR100546 | 0.0302 | TJR100498 | 0.1514 |
| TJR100542 | 0.0085 | TJR100522 | 0.0339 | TJR100535 | 0.1738 |
| TJR100528 | 0.0091 | TJR100534 | 0.0447 | TJR100512 | 0.1832 |
| TJR100541 | 0.0100 | TJR100533 | 0.0461 | TJR100538 | 0.1995 |
| TJR100513 | 0.0103 | TJR100508 | 0.0479 | TJR100503 | 0.2344 |
| TJR100507 | 0.0106 | TJR100499 | 0.0494 | TJR100502 | 0.3043 |
| TJR100520 | 0.0112 | TJR100536 | 0.0525 | TJR100530 | 0.3048 |
| TJR100516 | 0.0113 | TJR100519 | 0.0617 | TJR100518 | 0.5012 |
| TJR100532 | 0.0120 | TJR100547 | 0.0637 | TJR100509 | 0.5585 |
| TJR100543 | 0.0120 | TJR100496 | 0.0724 | TJR100501 | 0.7025 |
| TJR100531 | 0.0129 | TJR100521 | 0.0724 | TJR100504 | 5.7987 |
| TJR100739 | 4.0622 | TJR100741 | 0.0356 | TJR100742 | 0.0470 |
| TJR100816 | 0.0818 | TJR100817 | 0.0064 | TJR100818 | 0.0061 |
| TJR100819 | 0.0074 | TJR100820 | 0.4806 | TJR100821 | 0.0085 |
| TJR100822 | 0.0544 | TJR100823 | 0.0522 | TJR100824 | 0.0200 |
| TJR100825 | 0.0114 | | | | |

The results in Table 7 demonstrate that the siRNAs of the present disclosure had excellent on-target inhibitory activity against INHBE.

### Example 4. Multi-Concentration-Point Inhibitory Activity Against INHBE in Human Hepatoma Cell Line (Hep3B)

24 h prior to transfection, Hep3B cells were seeded into a 96-well plate at about 20,000 cells/well, with each well containing 100 µL of a culture medium. The cells were transfected with the samples using Lipofectamine RNAi MAX (ThermoFisher, 13778150) following the instruction manual of the product, with the final concentration gradients of the samples for transfection being 20 nM, 4 nM, 0.8 nM, 0.16 nM, 0.032 nM, 0.0064 nM, and 0.00128 nM. After 24 h of treatment, total cellular RNA extraction was performed using a high-throughput cellular RNA extraction kit (FG0417-L/FG0418-XL, magnetic bead method), followed by an RNA reverse transcription experiment (Takara, RR037A) and a quantitative real-time PCR (Thermo, 4444557) assay. The mRNA level of human INHBE was determined and calibrated on the basis of the GAPDH internal reference gene level.

The instruments involved in this experiment are shown in Table 8.

**Table 8. Experimental instruments**

| Experimental instrument | | |
|---|---|---|
| Name | Company | Cat. No./model |
| Nanodrop | Thermo | Nanodrop One |
| qPCR system | ABI | 7500 Fast |
| PCR | Life | Pro-Flex PCR system |

In the real-time quantitative PCR assay, a probe Q-PCR assay was used. Information about the primers is shown in Table 9.

**Table 9. Taqman primer information**

| Primer name | SEQ ID NO | Primer sequence |
|---|---|---|
| hINHBE-F | 493 | CAACAACCACCTGGCAATATG |
| hINHBE-R | 494 | ACATCAGCCAACCTGGAATAA |
| hINHBE-P | 495 | CTATGGGACCCAAATGGGCACTTTCT |
| hGAPDH-F | 496 | CCAGGTGGTCTCCTCTGACTTC |
| hGAPDH-R | 497 | GTGGTCGTTGAGGGCAATG |
| hGAPDH-P | 498 | ACAGCGACACCCACTCCTCCACCTT |

### Result analysis method

After the Q-PCR assay was completed, the corresponding Ct values were obtained based on the threshold automatically set by the system. The expression of a certain gene can be relatively quantified by comparing the Ct values. Comparing Ct refers to calculating the differences in gene expression by the differences from the Ct value of the internal reference gene, which is also referred to as 2-ΔΔCt, where ΔΔCt = [(Ct target gene in experimental group - Ct internal reference in experimental group) - (Ct target gene in control group - Ct internal reference in control group)]. Inhibition rate (%) = (1 - remaining level of target gene expression) × 100%.

The results are expressed relative to the remaining percentage of human INHBE mRNA expression in cells treated with the siRNA. The IC₅₀ value corresponds to the concentration of the siRNA used when the remaining percentage of INHBE mRNA expression is 50%.

The data in Table 10 demonstrate that the siRNAs of the present disclosure had high levels of on-target inhibitory activity against the INHBE gene in Hep3B cells.

**Table 10. Inhibitory activity of siRNAs against INHBE in Hep3B cells**

| Double strand code | IC₅₀ (nM) |
|---|---|
| TJR100532 | 0.5169 |
| TJR101194 | 0.7274 |
| TJR101675 | 0.3306 |
| TJR101677 | 0.9340 |
| TJR100817 | 0.1950 |
| TJR101198 | 0.1535 |
| TJR101676 | 0.1771 |
| TJR101678 | 0.2248 |
| TJR101679 | 0.1971 |
| TJR101197 | 0.1648 |
| TJR100818 | 0.1296 |
| TJR101200 | 0.1478 |
| TJR100821 | 0.1072 |
| TJR101199 | 0.1598 |
| TJR100819 | 0.1383 |

### Example 5. Multi-Concentration-Point Inhibitory Activity Against INHBE in Primary Human Hepatocytes (PHHs)

24 h prior to transfection, PHH cells were seeded into a 96-well plate at about 30,000 cells/well, with each well containing 100 µL of a culture medium. The cells were transfected with the samples using Lipofectamine RNAi MAX (ThermoFisher, 13778150) following the instruction manual of the product, with the final concentration gradients of the samples for transfection being 20 nM, 4 nM, 0.8 nM, 0.16 nM, 0.032 nM, 0.0064 nM, and 0.00128 nM. After 24 h of treatment, total cellular RNA extraction was performed using a high-throughput cellular RNA extraction kit (FG0417-L/FG0418-XL, magnetic bead method), followed by an RNA reverse transcription experiment (Takara, RR037A) and a quantitative real-time PCR (Thermo, 4444557) assay. The mRNA level of human INHBE was determined and calibrated on the basis of the GAPDH internal reference gene level.

The instruments, primer information, result analysis method, and calculation method for the remaining percentage of the target gene mRNA expression involved in this experiment were the same as those in Example 4.

The data in Table 11 demonstrate that the siRNAs of the present disclosure had high levels of on-target inhibitory activity against the INHBE gene in PHH cells.

**Table 11. Inhibitory activity of siRNAs of the present disclosure against INHBE in PHH cells**

| Sample No. | Remaining percentage of target gene mRNA expression (PHH) (mean, %) | | | | | | | PHH IC50 (nM) |
|---|---|---|---|---|---|---|---|---|
| | 20 | 4 | 0.8 | 0.16 | 0.032 | 0.0064 | 0.000128 | |
| TJR102203 | 16.71 | 22.92 | 24.57 | 35.03 | 50.85 | 84.67 | 90.42 | 0.0424 |
| TJR102204 | 15.10 | 12.08 | 13.69 | 21.07 | 46.10 | 78.89 | 112.22 | 0.0234 |

### Example 6. psiCHECK Validation of On-Target Levels

The siRNAs were subjected to an *in vitro* molecular-level simulation of on-target activity screening in HEK293A cells using 2 concentration gradients. The experimental process, experimental materials and instruments used, dilution process, and result analysis method were the same as those in Example 3.

The results in Table 12 demonstrate that the siRNAs of the present disclosure had good inhibitory effects on INHBE.

**Table 12. Inhibitory effects of siRNAs of the present disclosure on INHBE**

| No. | Remaining percentage of target gene mRNA expression (GSCM) (mean, %) | |
|---|---|---|
| | 0.31 nM | 0.08 nM |
| TJR100817 | 10.04 | 12.91 |
| TJR100820 | 51.21 | 85.92 |
| TJR100532 | 9.52 | 19.98 |
| TJR100533 | 21.03 | 62.60 |
| TJR101194 | 10.58 | 22.15 |
| TJR100534 | 20.99 | 40.91 |
| TJR102690 | 105.91 | 111.34 |
| TJR102691 | 49.22 | 78.22 |
| TJR102692 | 58.21 | 78.49 |
| TJR100543 | 12.73 | 28.61 |
| TJR102694 | 43.19 | 71.41 |
| TJR102695 | 15.91 | 25.01 |
| TJR102924 | 17.68 | 38.54 |
| TJR102925 | 25.81 | 59.61 |
| TJR102926 | 29.65 | 57.18 |
| TJR102927 | 17.68 | 38.54 |

### Example 7. psiCHECK Validation of On-Target Levels

The siRNAs were subjected to an *in vitro* molecular-level simulation of on-target activity screening in HEK293A cells using 9 concentration gradients. The experimental process, experimental materials and instruments used, dilution process, and result analysis method were all the same as those in Example 3.

The results in Table 13 demonstrate that in the results, > 20 suggests that the inhibition rate cannot reach 50% under the condition of the highest concentration of 20 nM in this example, and the siRNAs of the present disclosure had good inhibitory effects on INHBE.

**Table 13. Inhibitory effects of siRNAs of the present disclosure on INHBE**

| Double strand code | GSCM IC₅₀ (nM) |
|---|---|
| TJR102664 | 0.0107 |
| TJR102665 | 0.0391 |
| TJR102666 | 0.0421 |
| TJR102667 | 0.0093 |
| TJR102668 | 0.0680 |
| TJR102669 | >20 |
| TJR102670 | >20 |
| TJR102671 | >20 |
| TJR102673 | 0.0147 |
| TJR102675 | >20 |
| TJR102661 | 0.0262 |

### Example 8: Multi-Concentration-Point Inhibitory Activity Against INHBE in Human Hepatoma Cell Line (Huh7)

24 h prior to transfection, Huh7 cells were seeded into a 96-well plate at about 15,000 cells/well, with each well containing 100 µL of a culture medium. The cells were transfected with the samples using Lipofectamine RNAi MAX (ThermoFisher, 13778150) following the instruction manual of the product, with the final concentration gradients of the samples for transfection being 20 nM, 4 nM, 0.8 nM, 0.16 nM, 0.032 nM, 0.0064 nM, and 0.00128 nM. After 24 h of treatment, total cellular RNA extraction was performed using a high-throughput cellular RNA extraction kit (FG0417-L/FG0418-XL, magnetic bead method), followed by an RNA reverse transcription experiment (Takara, RR037A) and a quantitative real-time PCR (Thermo, 4444557) assay. The mRNA level of human INHBE was determined and calibrated on the basis of the GAPDH internal reference gene level.

The instruments, primer information, and result analysis method involved in this experiment were used with reference to Example 4.

The data in Table 14 demonstrate that the siRNAs and conjugates thereof all had high levels of on-target inhibitory activity against the INHBE gene in Huh7 cells.

**Table 14. Inhibitory activity of siRNA conjugates against INHBE in Huh7 cells**

| Sample No. | Remaining percentage of target gene mRNA expression (Huh7) (mean, %) | | | | | | | Huh7 IC₅₀ (nM) |
|---|---|---|---|---|---|---|---|---|
| | 20 | 4 | 0.8 | 0.16 | 0.032 | 0.0064 | 0.00128 | |
| TJR102154 | 20.46 | 30.29 | 32.23 | 50.85 | 91.17 | 100.81 | 103.41 | 0.1759 |
| TJR102152 | 25.82 | 37.74 | 51.25 | 76.27 | 98.76 | 109.51 | 108.45 | 0.9199 |

### Example 9. Multi-Concentration-Point Inhibitory Activity Against INHBE in Primary Monkey Hepatocytes (PCHs)

24 h prior to transfection, PCH cells were seeded into a 96-well plate at about 30,000 cells/well, with each well containing 100 µL of a culture medium. The cells were transfected with the samples using Lipofectamine RNAi MAX (ThermoFisher, 13778150) following the instruction manual of the product, with the final concentration gradients of the samples for transfection being 20 nM, 4 nM, 0.8 nM, 0.16 nM, 0.032 nM, 0.0064 nM, and 0.00128 nM. After 24 h of treatment, total cellular RNA extraction was performed using a high-throughput cellular RNA extraction kit (FG0417-L/FG0418-XL, magnetic bead method), followed by an RNA reverse transcription experiment (Takara, RR037A) and a quantitative real-time PCR (Thermo, 4444557) assay. The mRNA level of monkey INHBE was determined and calibrated on the basis of the GAPDH internal reference gene level.

The instruments involved in this experiment are shown in Table 7 of Example 4.

In the real-time quantitative PCR assay, a probe Q-PCR assay was used. Information about the primers is shown in Table 15.

**Table 15. Taqman primer information**

| Primer name | SEQ ID NO | Primer sequence |
|---|---|---|
| mkINHBE-F | 499 | GGTTGCATCTGACCAGTCGT |
| mkINHBE-R | 500 | GGCTGAAGTGGAGTCTGTGAC |
| mkINHBE-P | 501 | CCATTCCCTGGAGCCACACTCC |
| mkGAPDH-F | 502 | TCAAGATCGTCAGCAACGCC |
| mkGAPDH-R | 503 | ACAGTCTTCTGGGTGGCAGT |
| mkGAPDH-P | 504 | ACCAACTGCTTAGCACCCCTGGCCA |

The result analysis method and calculation method for the remaining percentage of the target mRNA expression were the same as those in Example 4.

The data in Table 16 demonstrate that the siRNA conjugates both had high levels of on-target inhibitory activity against the INHBE gene in PCH cells.

**Table 16. Inhibitory activity of siRNA conjugates against INHBE in PCH cells**

| Sample No. | Remaining percentage of target gene mRNA expression (PCH) (mean, %) | | | | | | | PCH IC₅₀ (nM) |
|---|---|---|---|---|---|---|---|---|
| | 20 | 4 | 0.8 | 0.16 | 0.032 | 0.0064 | 0.00128 | |
| TJR102154 | 58.66 | 51.47 | 47.38 | 50.20 | 72.01 | 96.98 | 85.05 | 0.0400 |
| TJR102152 | 39.63 | 46.38 | 49.75 | 43.37 | 49.77 | 77.88 | 104.56 | 0.0302 |

### Example 10. psiCHECK Validation of On-Target Levels

The siRNAs were subjected to an *in vitro* molecular-level simulation of on-target activity screening in HEK293A cells using 9 concentration gradients. The experimental process, experimental materials and instruments used, and the dilution process were the same as those in Example 3.

The data in Table 17 demonstrate that compared with the control sequence, the siRNAs of the present disclosure had higher levels of on-target inhibitory activity.

**Table 17. Inhibitory effects of siRNAs of the present disclosure on INHBE**

| No. | Remaining percentage of target gene mRNA expression (GSCM) (mean, %) | | | | |
|---|---|---|---|---|---|
| | 20.0000 nM | 5.0000 nM | 1.2500 nM | 0.3125 nM | 0.0781 nM |
| TJR102826 | 14.41 | 17.44 | 19.11 | 24.37 | 45.84 |
| TJR102827 | 25.19 | 28.93 | 31.12 | 53.33 | 83.20 |
| TJR102829 | 17.85 | 18.46 | 19.35 | 25.23 | 45.26 |
| TJR102831 | 6.64 | 6.96 | 8.31 | 10.10 | 19.55 |
| TJR102832 | 17.56 | 18.37 | 24.49 | 40.82 | 67.06 |
| TJR102833 | 12.69 | 13.77 | 17.10 | 31.10 | 67.98 |
| TJR102835 | 14.60 | 15.44 | 17.34 | 20.53 | 37.44 |
| TJR102836 | 17.97 | 24.05 | 31.00 | 54.19 | 87.56 |
| TJR102838 | 14.72 | 16.82 | 21.73 | 36.91 | 62.48 |

| No. | Remaining percentage of target gene mRNA expression (GSCM) (mean, %) | | | | GSCM IC50 (nM) |
|---|---|---|---|---|---|
| | 0.0195 nM | 0.0049 nM | 0.0012 nM | 0.0003 nM | |
| TJR102826 | 72.44 | 90.76 | 100.08 | 106.72 | 0.0592 |
| TJR102827 | 94.83 | 99.52 | 107.52 | 108.70 | 0.3732 |
| TJR102829 | 75.22 | 95.48 | 98.94 | 97.15 | 0.0618 |
| TJR102831 | 40.34 | 68.68 | 85.86 | 87.60 | 0.0128 |
| TJR102832 | 89.15 | 92.06 | 100.94 | 96.75 | 0.1881 |
| TJR102833 | 82.71 | 91.36 | 104.19 | 99.70 | 0.1401 |
| TJR102835 | 64.98 | 87.51 | 89.48 | 97.90 | 0.0403 |
| TJR102836 | 95.63 | 110.29 | 100.12 | 112.17 | 0.4017 |
| TJR102838 | 84.02 | 99.08 | 99.84 | 101.91 | 0.1451 |

### Example 11. psiCHECK Validation of On-Target Levels

The siRNAs were subjected to an *in vitro* molecular-level simulation of on-target activity screening in HEK293A cells using 9 concentration gradients. The experimental process, experimental materials and instruments used, dilution process, and result analysis method were all the same as those in Example 3. The data in Table 18 demonstrate that the siRNAs of the present disclosure had high levels of on-target inhibitory activity.

**Table 18. Inhibitory effects of siRNAs of the present disclosure on INHBE**

| No. | Remaining percentage of target gene mRNA expression (GSCM) (mean, %) | | | | |
|---|---|---|---|---|---|
| | 20.0000 nM | 5.0000 nM | 1.2500 nM | 0.3125 nM | 0.0781 nM |
| TJR102928 | 11.75 | 9.38 | 10.48 | 10.24 | 14.50 |
| TJR102930 | 20.98 | 25.42 | 32.42 | 43.86 | 78.61 |
| TJR102931 | 15.33 | 15.49 | 14.55 | 15.97 | 29.82 |
| TJR102932 | 18.91 | 18.89 | 20.33 | 22.85 | 38.52 |
| TJR102933 | 41.40 | 31.28 | 28.35 | 35.49 | 62.34 |
| TJR102320 | 12.05 | 9.97 | 9.26 | 13.29 | 26.60 |
| TJR100497 | 12.19 | 11.78 | 11.94 | 15.75 | 26.11 |
| TJR102843 | 8.87 | 10.72 | 23.37 | 43.37 | 73.43 |
| TJR102845 | 10.10 | 9.20 | 10.73 | 14.57 | 34.21 |
| TJR100500 | 13.40 | 14.57 | 17.36 | 24.63 | 42.23 |
| TJR102850 | 13.14 | 21.50 | 45.00 | 66.39 | 92.29 |
| TJR102852 | 11.97 | 12.73 | 19.56 | 39.66 | 68.04 |

| No. | Remaining percentage of target gene mRNA expression (GSCM) (mean, %) | | | | GSCM IC50 (nM) |
|---|---|---|---|---|---|
| | 0.0195 nM | 0.0049 nM | 0.0012 nM | 0.0003 nM | |
| TJR102928 | 36.20 | 73.73 | 92.37 | 104.37 | 0.0112 |
| TJR102930 | 104.07 | 100.35 | 104.36 | 103.41 | 0.2464 |
| TJR102931 | 64.84 | 96.63 | 108.04 | 110.54 | 0.0317 |
| TJR102933 | 87.20 | 96.62 | 91.68 | 99.75 | 0.1220 |
| TJR102322 | 36.34 | 65.65 | 92.11 | 84.92 | 0.0107 |
| TJR102320 | 60.24 | 88.93 | 98.92 | 101.96 | 0.0279 |
| TJR100497 | 62.96 | 93.06 | 106.38 | 106.76 | 0.0291 |
| TJR102843 | 83.06 | 91.65 | 92.01 | 90.98 | 0.2453 |
| TJR102845 | 62.27 | 91.74 | 99.71 | 108.97 | 0.0339 |
| TJR100500 | 78.54 | 88.19 | 100.00 | 106.62 | 0.0603 |
| TJR102850 | 99.03 | 101.08 | 109.05 | 102.56 | 0.8513 |
| TJR102852 | 91.16 | 104.41 | 108.90 | 113.74 | 0.1766 |

## Claims

1. An siRNA, comprising a sense strand and an antisense strand forming a doublestranded region, wherein
the sense strand comprises at least 15 contiguous nucleotides and differs from any one of the nucleotide sequences of SEQ ID NOs: 1, 2, 3, 4, 5, 6, 7, 8, and 9 by no more than 3 nucleotides; and
the antisense strand comprises at least 15 contiguous nucleotides and differs from any one of the nucleotide sequences of SEQ ID NOs: 10, 11, 12, 13, 14, 15, 16, 17, and 18 by no more than 3 nucleotides.

2. The siRNA according to claim 1, wherein the sense strand comprises at least 17 contiguous nucleotides that differ from any one of the nucleotide sequences of SEQ ID NOs: 1 to 9 by no more than 3 nucleotides; the antisense strand comprises at least 17 contiguous nucleotides that differ from any one of the nucleotide sequences of SEQ ID NOs: 10 to 18 by no more than 3 nucleotides;
preferably, the sense strand comprises at least 19 contiguous nucleotides that differ from any one of the nucleotide sequences of SEQ ID NOs: 1 to 9 by no more than 3 nucleotides, preferably, by no more than 1 nucleotide, and/or
the antisense strand comprises at least 21 contiguous nucleotides that differ from any one of the nucleotide sequences of SEQ ID NOs: 10 to 18 by no more than 3 nucleotides, preferably, by no more than 1 nucleotide.

3. The siRNA according to claim 1 or 2, comprising or selected from any one of the following groups:
group 1), a sense strand set forth in SEQ ID NO: 1 and an antisense strand set forth in SEQ ID NO: 10;
group 2), a sense strand set forth in SEQ ID NO: 2 and an antisense strand set forth in SEQ ID NO: 11;
group 3), a sense strand set forth in SEQ ID NO: 3 and an antisense strand set forth in SEQ ID NO: 12;
group 4), a sense strand set forth in SEQ ID NO: 4 and an antisense strand set forth in SEQ ID NO: 13;
group 5), a sense strand set forth in SEQ ID NO: 5 and an antisense strand set forth in SEQ ID NO: 14;
group 6), a sense strand set forth in SEQ ID NO: 6 and an antisense strand set forth in SEQ ID NO: 15;
group 7), a sense strand set forth in SEQ ID NO: 7 and an antisense strand set forth in SEQ ID NO: 16;
group 8), a sense strand set forth in SEQ ID NO: 8 and an antisense strand set forth in SEQ ID NO: 17; and
group 9), a sense strand set forth in SEQ ID NO: 9 and an antisense strand set forth in SEQ ID NO: 18.

4. The siRNA according to any one of claims 1 to 3, wherein at least one nucleotide in the sense strand and/or the antisense strand is a modified nucleotide.

5. The siRNA according to claim 4, wherein three contiguous nucleotides in the sense strand are 2'-fluoro-modified nucleotides; preferably, in the direction from the 5' end to the 3' end, nucleotides at positions 7, 8, and 9 of the sense strand are each independently a 2'-fluoro-modified nucleotide, and in the direction from the 5' end to the 3' end, nucleotides at positions 2, 6, 12, and 14 of the antisense strand are each independently a 2'-fluoro-modified nucleotide.

6. The siRNA according to any one of claims 1 to 5, wherein at least one phosphodiester group in the sense strand and/or the antisense strand is a phosphodiester group with a modification group, preferably a phosphorothioate diester group.

7. The siRNA according to claim 6, wherein the phosphorothioate diester group is present in at least one of the following positions selected from the group consisting of:
a position between the 1st and 2nd nucleotides at the 5' end of the sense strand;
a position between the 2nd and 3rd nucleotides at the 5' end of the sense strand;
a position between the 1st and 2nd nucleotides at the 3' end of the sense strand;
a position between the 2nd and 3rd nucleotides at the 3' end of the sense strand;
a position between the 1st and 2nd nucleotides at the 5' end of the antisense strand;
a position between the 2nd and 3rd nucleotides at the 5' end of the antisense strand;
a position between the 1st and 2nd nucleotides at the 3' end of the antisense strand;
a position between the 2nd and 3rd nucleotides at the 3' end of the antisense strand;
preferably, the sense strand and/or the antisense strand comprise a plurality of phosphorothioate diester groups.

8. The siRNA according to any one of claims 1 to 7, wherein the sense strand comprises the nucleotide sequence set forth in any one of SEQ ID NO: 227 to SEQ ID NO: 242; and/or the antisense strand comprises the nucleotide sequence set forth in any one of SEQ ID NO: 251 to SEQ ID NO: 266.

9. An siRNA conjugate, comprising the siRNA according to any one of claims 1 to 8, and a targeting ligand linked to an end of the siRNA,
wherein preferably, the targeting ligand targets the liver;
more preferably, the targeting ligand binds to an asialoglycoprotein receptor (ASGPR), and is selected from the group consisting of one or more of N-acetyl-galactosamine, N-trifluoroacetylgalactosamine, N-propionylgalactosamine, N-n-butyrylgalactosamine, and N-isobutyrylgalactosamine;
yet more preferably, the targeting ligand has the following structure: 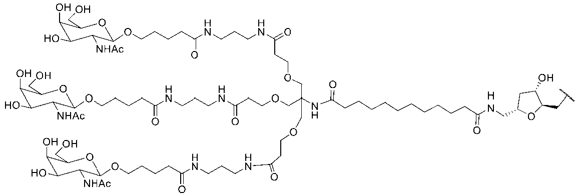 or

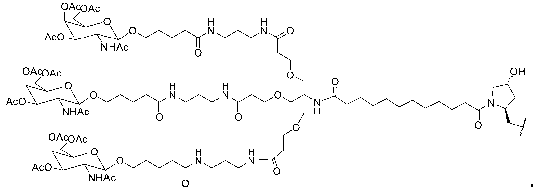

10. The siRNA conjugate according to claim 9, wherein the targeting ligand is linked to the 3' end or the 5' end of the sense strand or the antisense strand in the siRNA via a phosphorothioate diester group or a phosphodiester group; preferably, the targeting ligand is linked to the 3' end of the sense strand of the siRNA.

11. The siRNA conjugate according to claim 10, wherein the sense strand comprises the nucleotide sequence set forth in any one of SEQ ID NO: 243 to SEQ ID NO: 250; and/or the antisense strand comprises the nucleotide sequence set forth in any one of SEQ ID NO: 267 to SEQ ID NO: 274.

12. A pharmaceutical composition, comprising the siRNA according to any one of claims 1 to 8 or the siRNA conjugate according to any one of claims 9 to 11, and a pharmaceutically acceptable carrier.

13. Use of the siRNA according to any one of claims 1 to 8 or the siRNA conjugate according to any one of claims 9 to 11 in the manufacture of a medicament for reducing the expression of inhibin βE (INHBE).

14. A method for reducing the expression of inhibin βE (INHBE) in a subject in need thereof, comprising administering to the subject an effective amount or an effective dose of the siRNA according to any one of claims 1 to 8, or the siRNA conjugate according to any one of claims 9 to 11, or the pharmaceutical composition according to claim 12.

15. A method for treating and/or preventing a disease in a subject in need thereof, comprising administering to the subject an effective amount or an effective dose of the siRNA according to any one of claims 1 to 8, or the siRNA conjugate according to any one of claims 9 to 11, or the pharmaceutical composition according to claim 12, wherein preferably, the disease is selected from the group consisting of metabolic disorders; more preferably, the disease is selected from the group consisting of metabolic syndrome (MS), a cardiovascular disease, obesity, hepatitis, and a renal disease.

16. A method for delivering an siRNA that inhibits the expression and/or replication of INHBE to the liver in a subject in need thereof *in vivo,* comprising administering to the subject the siRNA according to any one of claims 1 to 8, or the siRNA conjugate according to any one of claims 9 to 11, or the pharmaceutical composition according to claim 12.

17. A method for preparing an siRNA or an siRNA conjugate, comprising synthesizing the siRNA according to any one of claims 1 to 8, or the siRNA conjugate according to any one of claims 9 to 11, or the pharmaceutical composition according to claim 12.
